# EUROPEAN PATENT SPECIFICATION

(11) **EP 1 109 559 B1**
(45) Date of publication and mention of the grant of the patent: **26.10.2005**
(21) Application number: 99936952.3
(22) Date of filing: 13.08.1999
(51) Int. Cl.: A61K 31/505, A61P 9/00

(54) **INHIBITION OF PATHOGENIC PROCESSES RELATED TO TISSUE TRAUMA**
HEMMUNG VON MIT GEWEBETRAUMA VERBUNDENEN PATHOGENEN PROZESSEN
INHIBITION DE PROCEDES LIES A DES LESIONS TISSULAIRES

(30) Priority: 13.08.1998 IL 12579098; 01.06.1999 US 137145 P
(43) Date of publication of application: 27.06.2001
(73) Proprietor: HADASIT MEDICAL RESEARCH SERVICES & DEVELOPMENT CO., LTD., Jerusalem 91120 (IL); Agricultural Research Organization, 50250 Beit Dagan (IL)
(72) Inventor: PINES, Mark, 76308 Rehovot (IL); VLODAVSKY, Israel, 90805 Mevaseret Zion (IL); NAGLER, Arnon, 74381 Jerusalem (IL); HAZUM, Eli, Rehovot 76308 (IL)
(74) Representative: Hartley, Andrew Philip
(86) International application number: PCT/IL1999/000440
(87) International publication number: WO 2000/009070

(56) References cited:
- WO-A-96/06616
- DE-A- 19 951 702
- US-A- 3 320 124
- US-A- 4 340 596
- US-A- 5 449 678
- US-A- 5 852 024
- US-A- 6 028 075
- PATENT ABSTRACTS OF JAPAN vol. 1997, no. 07, 31 July 1997 (1997-07-31) & JP 09 071586 A (YAMANOUCHI PHARMACEUT CO LTD), 18 March 1997 (1997-03-18)
- NAGLER ET AL.: 'Halofuginone-an inhibitor of collagen type I synthesis-prevents postoperative formation of abdominal adhesions' ANNALS OF SURGERY vol. 227, no. 4, 1998, pages 575 - 582, XP000911972

## Description

### FIELD OF THE INVENTION

The present invention relates to the inhibition of pathogenic processes associated with tissue trauma by regulating, at the molecular level, the extracellular matrix economy. More particularly, the present invention relates to compositions containing a quinazolinone derivative which are useful for such regulation, especially the quinazolinone Halofuginone, and other molecules which may exert effects through the same mechanisms at the molecular level.

In particular, it is now disclosed that these molecules are potent inhibitors of nuclear factor κB (NF-κB) transcription, thereby preventing the damaging cascade of pathogenic processes that is initiated by trauma, without subverting the normal repair mechanisms.

### BACKGROUND OF THE INVENTION

Degradation and remodeling of the ECM are essential processes for normal repair after tissue trauma. However, these mechanisms are also involved in of a number of different pathological processes, including the formation of adhesions, hepatic fibrosis and cirrhosis, the formation of keloids and hypertrophic scars, and pulmonary fibrosis. All of these pathophysiological processes are abnormal responses to tissue trauma. Yet, each such process represents a different type of different fibrosis, involving very different types of tissues, with potentially different underlying mechanisms.

The physiological response to tissue trauma is a complex process involving multiple factors including cell migration and replication, turnover of extracellular matrix (ECM) components and changes to the cellular microenvironment. Essentially, such a response involves the repair or replacement of damaged tissues. The precise nature of such repair or replacement depends upon the tissues involved, although all such processes involve certain basic principles. The normal and necessary repair of any tissue after any trauma requires the coordination of a wide array of factors by regulated gene expression.

The pathophysiological response to the tissue trauma may differ in these tissues as well, but often results in the formation of adhesions or other types of abnormal tissues which do not duplicate the functionality of the original organ tissue, so that the repair of tissue trauma does not lead to a complete restoration of organ capacity and function.

One example of a fibrotic process which results from pathophysiological responses to tissue trauma is cardiac fibrosis.

Cardiac fibrosis has a number of causes, which lead to the deposition of fibrotic tissue. As the deposition of such fibrotic tissue increases, the ability of the heart to function decreases, leading to disability and eventually death of the patient. The formation of fibrotic tissue in the heart is characterized by the deposition of abnormally large amounts of extracellular matrix components, including collagen, as well as other matrix proteins. Therefore, the cardiac fibrotic process needs to be inhibited in order to prevent damage to the cardiac tissue and hence to the ability of the heart to function.

Unfortunately, currently available treatments to inhibit various abnormal responses to tissue trauma, such as the formation and growth of keloids and hypertrophic scars, cardiac fibrosis and other types of fibrotic disease processes, are not completely successful. For example, surgery can be used to reduce the size or extent of the lesion, while physical pressure can be used to reduce the size and extent of keloids and hypertrophic scars, as well as to prevent their initial formation [D.D. Datubo-Brown, *Brit. J. Plas. Surg.,* Vol 43, p. 70-77, 1990]. However, neither treatment can prevent the lesion from recurring, and surgery especially carries a risk of increased morbidity.

Other forms of treatment include the administration of corticosteroids. For example, triamcinolone appears to reduce the size of keloids and hypertrophic scars by increasing the rate of collagen degradation [Rockwell, W.B. *et al*., *Plastic and Recon. Surg.,* Vol. 84, p. 827-835, 1989]. However, the side effects of such medications are potentially dangerous and are not universally successful. Other treatments, such as radiation, also showed variable effectiveness and are associated with other potential side effects [Rockwell, W.B. *et al*., *Plastic and Recon. Surg*., Vol. 84, p. 827-835, 1989]. Thus, clearly improved treatments for these diseases, which are associated with pathophysiological fibrotic processes, are required.

As noted above, collagen synthesis and deposition plays an important role in keloid and hypertrophic scar formation, and in the formation of adhesions, as well as in the cell hyperproliferation associated with psoriasis and in the many different forms of pathological fibrosis such as cardiac fibrosis, pulmonary fibrosis and hepatic fibrosis. The synthesis of collagen is also involved in a number of other pathological conditions, particularly those associated with primary or secondary fibrosis. The crucial role of collagen in fibrosis has prompted attempts to develop drugs that inhibit the accumulation of collagen [K.I. Kivirikko, *Annals of Medicine,* Vol. 25, pp. 113-126 (1993)].

However, the deposition of ECM components, such as collagen, is currently believed to also be important for healing of the wound, as well as for general maintenance of the structure of the tissues. Indeed, the prior art teaches that the strength of the healing wound is ultimately dependent upon collagen deposition [Haukipuro, K., *et al*., *Ann. Surg.,* Vol. 213, p. 75-80 , 1991]. Thus, according to the prior art, collagen deposition must be present at a sufficient level to give the healing wound strength and support, yet not at such a high level to cause the formation of scars.

Furthermore, according to the prior art, simply abolishing collagen synthesis would be expected to have highly deleterious side effects. Unfortunately, certain medicaments which did abolish collagen synthesis, such as general inhibitors of collagen formation, were examined for their effect on collagen-dependent processes such as tumor growth, and were found to inhibit tumor growth in mice but proved too toxic for long-term safe administration. Thus, currently available inhibitors of collagen synthesis and deposition which were tested for their effects on fibrotic and/or collagen-related conditions, were found to be unsuitable for the treatment of malignancies and other collagen dependent or related disease conditions, such as the fibrotic diseases which were described above.

In addition, many other available inhibitors of collagen synthesis and deposition, although not examined for their effects on various fibrotic processes, are generally undesirable because they lack specificity for the collagen metabolic pathway. Thus, many currently available drugs have deleterious side effects.

For example, cytotoxic drugs have been used in an attempt to slow the proliferation of collagen-producing fibroblasts [J.A. Casas, *et al*., *Ann. Rhem. Dis.,* Vol. 46, p. 763 (1987)], such as colchicine, which slows collagen secretion into the extracellular matrix [D. Kershenobich, *et al*., *N. Engl. J. Med.,* Vol. 318, p. 1709 (1988)]. Other drugs act as inhibitors of key collagen metabolism enzymes [K. Karvonen, *et al*., *J. Biol Chem*., Vol. 265, p. 8414 (1990); C.J. Cunliffe, *et al*., *J. Med*. *Chem.,* Vol. 35, p.2652 (1992)]. However, none of these inhibitors have specific effects for the metabolism and deposition of specific types of collagen. Also, these drugs may interfere with the biosynthesis of other vital collagenous molecules, such as Clq in the classical complement pathway, acetylcholine esterase of the neuro-muscular junction endplate, conglutinin and pulmonary surfactant apoprotein. Such interference and lack of specificity could have potentially serious adverse effects.

Other drugs which can inhibit collagen synthesis, such as nifedipine and phenytoin, inhibit synthesis of other proteins as well, thereby non-specifically blocking the collagen biosynthetic pathway [T. Salo, *et al., J. Oral Pathol. Med*., Vol. 19, p. 404 (1990)]. Again, the lack of specificity significantly reduces the clinical use of these drugs, because the non-specific inhibition of protein synthesis can result in adverse side-effects when the drug is administered to the patient.

Indeed, clinically available anti-fibrotic drugs, including the collagen cross-linking inhibitors such as beta-amino-propionitrile discussed previously, are also non-specific. Unfortunately, the lack of specificity of these collagen cross-linking inhibitors ultimately results in severe side effects after prolonged use. These side effects include lathritic syndrome, as well as disrupted elastogenesis. The latter side effect is a result of the disruption of cross-linking of elastin, another fibrous connective tissue protein. In addition, the collagen cross-linking inhibitory effect of these drugs is secondary, so that collagen must first be overproduced before degradation by collagenase. Thus, a type-specific inhibitor of the synthesis of collagen itself is clearly required.

Such a type-specific collagen synthesis inhibitor is disclosed in U.S. Patent No. 5,449,678 for the treatment of certain fibrotic conditions such as scleroderma and Graft Versus Host Disease. Both of these conditions are associated with excessive collagen deposition, which can be inhibited by Halofuginone. This specific inhibitor is a composition with a pharmaceutically effective amount of a pharmaceutically active compound of a formula: 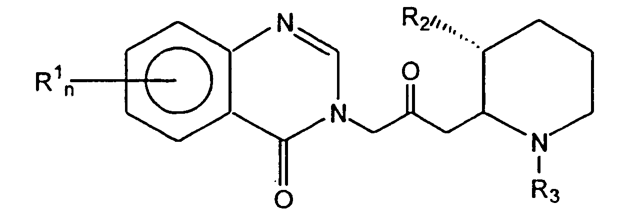 wherein:
R₁ is a member of the group consisting of hydrogen, halogen, nitro, benzo, lower alkyl, phenyl and lower alkoxy;
R₂ is a member of the group consisting of hydroxy, acetoxy and lower alkoxy; and
R₃ is a member of the group consisting of hydrogen and lower alkenoxy-carbonyl. Of this group of compounds, Halofuginone has been found to be particularly effective for such treatment.

PCT Patent Application No. WO 96/06616 further discloses that these compounds are able to effectively treat restenosis by preventing the proliferation of vascular smooth muscle cells. Restenosis is characterized by smooth muscle cell proliferation and extracellular matrix accumulation within the lumen of affected blood vessels in response to a vascular injury [Choi *et al*., *Arch. Surg.,* Vol. 130, p. 257-261 (1995)]. One hallmark of such smooth muscle cell proliferation is a phenotypic alteration, from the normal contractile phenotype to a synthetic one. Type I collagen has been shown to support such a phenotypic alteration, which can be blocked by Halofuginone *[Choi et al*., *Arch. Surg.,* Vol. 130, p. 257-261 (1995); PCT Patent Application No. 96/06616]. Thus, Halofuginone can prevent such abnormal redifferentiation of smooth muscle cells after vascular injury by blocking the synthesis of type I collagen. Other *in vitro* studies show that Halofuginone can also inhibit the proliferation of 3T3 fibroblast cells [U.S. Patent No. 5,449,678].

However, the process of restenosis differs from cardiac fibrosis. Furthermore, cardiac tissue is generally different than the tissue of other organs. In particular, cardiac tissue must maintain the ability to function as a single muscle according to a wave of electrical activity in order to pump blood effectively. Therefore, the heart must maintain a high level of functionality, in contrast to an organ such as the liver for example, which can be significantly compromised and still provide the required level of function in order to maintain the body. Thus, any amount of cardiac fibrosis is detrimental to the functioning of the heart, such that treatments which would be suitable for other organs would not be expected to be suitable for treating and/or preventing cardiac fibrosis.

Furthermore, the *in vitro* action of Halofuginone does not always predict its *in vivo* effects. For example, Halofuginone inhibits the synthesis of collagen type I in bone chrondrocytes *in vitro,* as demonstrated in U.S. Patent No. 5,449,678. However, chickens treated with Halofuginone were not reported to have an increased rate of bone breakage, indicating that the effect is not seen *in vivo.* Thus, the exact behavior of Halofuginone *in vivo* cannot always be predicted from *in vitro* studies.

Indeed, the initial discovery of the ability of Halofuginone to successfully treat several different disease states was completely serendipitous. Halofuginone has been shown to be effective for these disease states by trial-and-error, since the precise underlying mechanism of action of Halofuginone was not known. Such a lack of knowledge, coupled with the inability to completely predict the *in vivo* behavior of Halofuginone from its *in vitro* effects, has limited the development of new agents for these pathophysiological conditions.

The elucidation of the underlying mechanism of the action(s) of Halofuginone would enable new and potentially even more effective treatments to be developed. Furthermore, such treatments could be designed to precisely pinpoint the molecular targets of Halofuginone and other quinazolinone derivatives, thereby potentially reducing the unwanted side effects of treatment. Such treatments could also regulate the overall extracellular matrix economy, and thus lead to the amelioration of many different pathological conditions associated with disturbances in this economy.

There is thus a widely recognized unmet medical need for specific effectors capable of regulating the extracellular matrix economy, whose mechanism of action includes targeted intervention at the transcriptional or other molecular level, such that a specific effect on the pathological responses to tissue trauma is determined by a precise intervention at a particular molecular target, which could therefore act as an inhibitor of tumor growth, progression and metastasis, which is particularly effective *in vivo*, substantially without adversely affecting other physiological processes, and which is able to inhibit angiogenesis and the deposition of collagen, which is able to selectively induce apoptosis in tumor cells, and which is able to inhibit a variety of fibrotic disease processes, including cardiac fibrosis, in a specific and focused manner, such that the treatment does not result in untoward side effects.

### SUMMARY OF THE INVENTION

It is now disclosed that the ability of the compositions according to the present invention to prevent the pathogenic aspects of tissue trauma while preserving normal tissue repair mechanisms, is based on the fact that these molecules abrogate the cascade of damage initiated by tissue trauma, while maintaining the proper, healthy extracellular matrix economy.

According to one aspect of the invention, Halofuginone has been shown to be effective for the coordination or co-regulation of multiple genes whose activity is not known to be co-regulated. Such co-regulation is specific, in that certain genes which are known to be linked to the regulated genes in other systems are not co-regulated by Halofuginone. Indeed, such specific co-regulation indicates a common, underlying mechanism for all of these effects of quinazolinone derivatives such as Halofuginone.

According to another aspect of the invention, Halofuginone causes a panoply of effects which were not previously known to be related and which include:
a) decreasing the expression of collagen type I, in particular by inhibiting the expression of the collagen α1(I) gene;
b) decreasing the release of the cytokines IL-1β and TNFα, and inhibiting the transcription of NF-κB;
c) inhibiting collagenase type IV production;
d) promotion of activity of *cKrox* transcription factor;
e) decreasing the expression of the collagen chaperone heat shock protein HSP47 gene in parallel to the inhibition of the expression of the collagen α1(I) gene; and
f) lack of affect on the expression of TGFβ.

According to the present invention it is now disclosed that the underlying mechanism of action of Halofuginone and related quinolinones in the inhibition of pathogenic responses to tissue trauma involves the regulation of the extracellular matrix economy at the molecular level. Such regulation involves at least the following factors: inhibition of expression of collagen α1(I) gene, inhibition of transcription of NF-κB and inhibition of collagenase type IV production. Another important factor in the efficacy of regulation by Halofuginone is the promotion of the activity of the *cKrox* transcription factor.

NF-κB has become the focus of intense interest, as it has been shown to become activated in cells through a wide variety of stimulating factors which are associated with stress or injury. NF-κB has been shown to be induced through a large number of factors, such as IL-1β (interleukin-1β) and tumor necrosis factor α (TNFα) [Mercurio and Manning; *Curr. Op. in Cell Biol*., **11**:226-232, 1999]. Many inflammatory factors have also been shown to induce NF-κB, such that a wide variety of induction mechanisms are considered to converge on this particular target. Thus, the effect of Halofuginone on the inhibition of NF-κB expression may indicate at least one feature of the mechanism for inducing the effect of Halofuginone on the inhibition of fibrotic processes, while regulating and maintaining the physiologically normal and desirable extracellular matrix economy.

The *cKrox* transcription factor for the expression of the collagen α1(I) gene is a novel zinc finger-containing transcription factor which binds to the α1(I) and α2(I) collagen gene promoters, and was shown to repress transcription of the α1(I) procollagen promoter (Widom R L; Culic I; Lee J Y; Korn J H; *GENE,* (1997 Oct 1) **198**:407-20). As described in further detail in the examples below, Halofuginone, in turn, was shown to potentiate the effect of *cKrox* and thus to potentiate inhibition of collagen synthesis.

As described above, additional factors in the ability of Halofuginone to regulate the extracellular matrix economy may include a decrease in the release of the cytokines IL-1β and TNFα, and a decrease in the expression of the collagen chaperon HSP47. Concomitant with all of these actual effects on the extracellular matrix economy is a lack of any effect on the expression of TGFβ. Thus, clearly the regulation of the extracellular matrix economy by Halofuginone, while maintaining a healthy extracellular matrix, is specific for a particular underlying mechanism.

Some of these molecular targets of Halofuginone and other molecules of its class, such as the inhibition of the tumor marker H19 gene expression, as well as the overall regulation of ECM (extracellular matrix) deposition and remodeling, are likely to either be secondary targets for the mechanism of action of Halofuginone, or to only be indirectly inhibited by Halofuginone. Indeed, these inhibitory effects may in turn be related to the potentiation of the *cKrox* transcriptional factor, or to the regulation of the other mechanisms described above.

In any case, all of these mechanisms are related to the regulation of the "extracellular matrix economy". Regulation is not merely inhibition of all processes related to the extracellular matrix turnover and collagen deposition, since Halofuginone was previously shown by the inventors to inhibit excessive collagen deposition without interfering with basal levels of collagen expression necessary for wound repair as shown in US Patent 5,852,024.

The term "extracellular matrix economy" is intended to indicate the constellation of processes relating to the synthesis, deposition and maintenance of the extracellular matrix and related tissue structures. The proper regulation of the extracellular matrix economy leads to the inhibition of the pathological response to tissue trauma, such that all of the potential targets for the action of Halofuginone and other effectors are able to prevent such pathological responses substantially without inhibiting or altering other desirable physiological activity.

Furthermore, these specific effects of Halofuginone, and other compounds which achieve regulation of the extracellular matrix economy, are useful for the treatment of various diseases related to ECM deposition and other aspects of the ECM economy, as described in greater detail below. For example and unexpectedly, it has been found, as described in the examples below, that Halofuginone can inhibit the pathophysiological process of cardiac fibrosis *in vivo.*

According to a first aspect of the present invention, there is provided a method of manufacturing a medicament for treating cardiac fibrosis, comprising the step of placing a pharmaceutically effective amount of a compound in a pharmaceutically acceptable carrier, the compound being a member of a group having a formula: 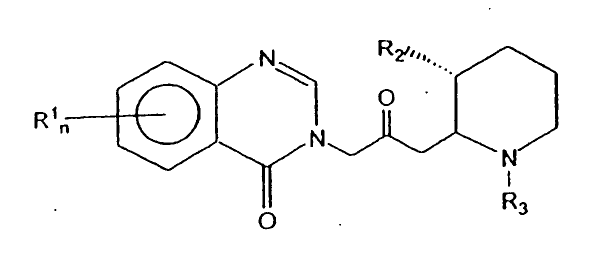 wherein:
R₁ is a member of the group consisting of hydrogen, halogen, nitro, benzo, lower alkyl, phenyl, and lower alkoxy; R₂ is a member of the group consisting of hydroxy, acetoxy and lower alkoxy; R₃ is a member of the group consisting of hydrogen and lower alkenoxy-carbonyl; and *n* is either 1 or 2. Pharmaceutically acceptable salts thereof are also included.

Another aspect of the present invention is the use of a compound having the formula: 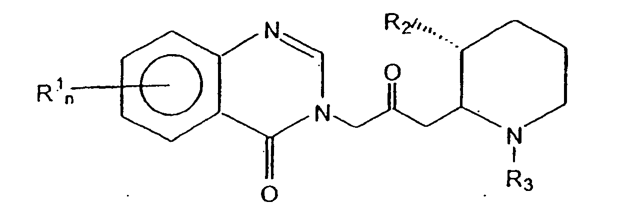 wherein:
R₁ is a member of the group consisting of hydrogen, halogen, nitro, benzo, lower alkyl, phenyl, and lower alkoxy; R₂ is a member of the group consisting of hydroxy, acetoxy and lower alkoxy; R₃ is a member of the group consisting of hydrogen and lower alkenoxy-carbonyl; and *n* is either 1 or 2; or of a pharmaceutically acceptable salt of such a compound; for the manufacture of a medicament for treating cardiac fibrosis.

In accordance with a third aspect of the invention, there is provided a method of manufacturing a medicament for preventing cardiac fibrosis, comprising the step of placing a pharmaceutically effective amount of a compound in a pharmaceutically acceptable carrier, the compound being a member of a group having a formula: 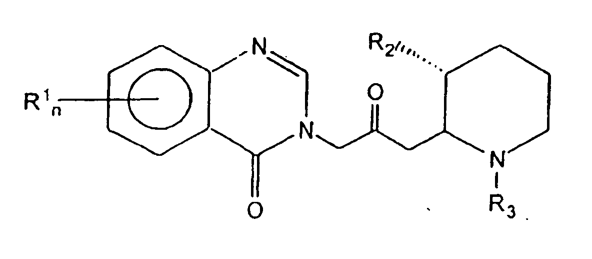 wherein:
R₁ is a member of the group consisting of hydrogen, halogen, nitro, benzo, lower alkyl, phenyl, and lower alkoxy; R₂ is a member of the group consisting of hydroxy, acetoxy, and lower alkoxy; R₃ is a member of the group consisting of hydrogen and lower alkenoxy-carbonyl; and *n* is either 1 or 2; and pharmaceutically acceptable salts thereof.

In accordance with a fourth aspect of the invention, there is provided use of a compound having a formula: 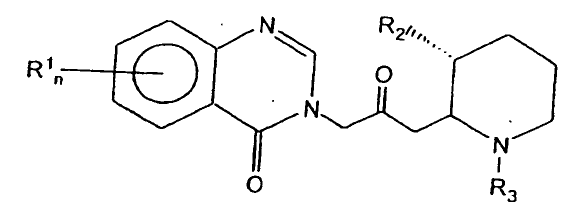 wherein:
R₁ is a member of the group consisting of hydrogen, halogen, nitro, benzo, lower alkyl, phenyl, and lower alkoxy; R₂ is a member of the group consisting of hydroxy, acetoxy and lower alkoxy, R₃ is a member of the group consisting of hydrogen and lower alkenoxy-carbonyl; and *n* is either 1 or 2; or of a pharmaceutically acceptable salt of such a compound; for the manufacture of a medicament for substantially preventing cardiac fibrosis.

According to preferred embodiments of the present invention, the compound is Halofuginone. Hereinafter, the term "Halofuginone" is defined as a compound having a formula: 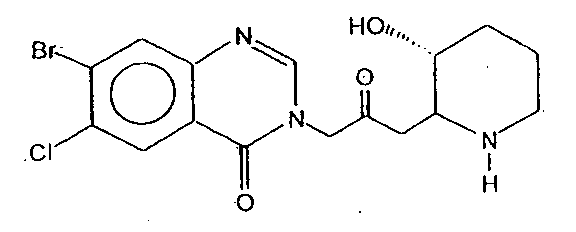 and pharmaceutically acceptable salts thereof. The composition preferably includes a pharmaceutically acceptable carrier for the compound.

Preferably, all of the compounds referred to hereinabove can be either the compound itself as described by the formula, and/or pharmaceutically acceptable salts thereof.

### BRIEF DESCRIPTION OF THE DRAWINGS

The invention is herein described, by way of example only, with reference to the accompanying drawings, wherein:
FIG. 1 illustrates certain exemplary aspects of the extracellular matrix economy;
FIG. 2 illustrates the dose-dependent inhibition of type IV collagenase activity in T50 bladder carcinoma cell cultures in the presence of Halofuginone;
FIG. 3 illustrates the inhibition of the expression of the H19 gene in the RT112 and 5376 bladder carcinoma cell lines by Halofuginone;
FIG. 4 shows a Northern blot with the effect of Halofuginone on Integrin αᵥ chain expression;
FIG. 5 shows the effect of Haiofuginone on β subunit expression as determined by RT-PCR;
FIG. 6 illustrates the effect of Halofuginone on ventricular collagen volume fraction (CVF) in rat heart;
FIG. 7 illustrates the effect of Halofuginone on collagen a 1(I) gene expression in rat heart; and
FIG. 8 illustrates the effect of Halofuginone on TGF-β expression in rat heart. 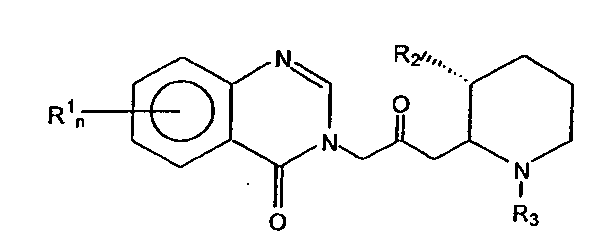
wherein:
R₁ is a member of the group consisting of hydrogen, halogen, nitro, benzo, lower alkyl, phenyl, and lower alkoxy; R₂ is a member of the group consisting of hydroxy, acetoxy and lower alkoxy, R₃ is a member of the group consisting of hydrogen and lower alkenoxyl-carbonyl: and *n* is either 1 or 2. Pharmaceutically acceptable salts thereof are also included, for preparing a pharmaceutical composition for treating cardiac fibrosis.

According to other embodiments of the present invention, there is provided a composition for substantially preventing cardiac fibrosis, comprising a pharmaceutically effective amount of a compound in combination with a pharmaceutically acceptable carrier, the compound being a member of a group having a formula: 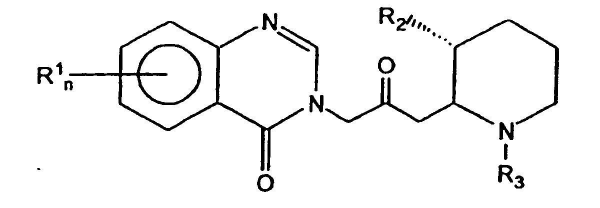 wherein:
R₁ is a member of the group consisting of hydrogen, halogen, nitro, benzo, lower alkyl, phenyl, and lower alkoxy; R₂ is a member of the group consisting of hydroxy, acetoxy, and lower alkoxy; R₃ is a member of the group consisting of hydrogen and lower alkenoxy-carbonyl; and *n* is either 1 or 2; and pharmaceutically acceptable salts thereof.

According to further preferred embodiments of the present invention, the compound is preferably Halofuginone. Hereinafter, the term "Halofuginone" is defined as a compound having a formula: 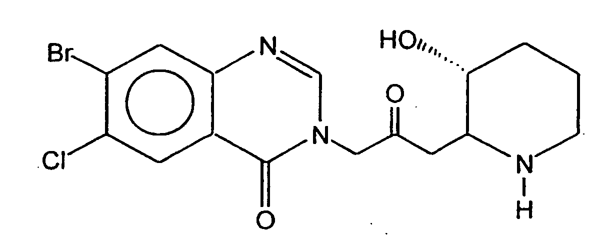 and pharmaceutically acceptable salts thereof. The composition preferably includes a pharmaceutically acceptable carrier for the compound.

Preferably, all of the compounds referred to hereinabove can be either the compound itself as described by the formula, and/or pharmaceutically acceptable salts thereof.

### BRIEF DESCRIPTION OF THE DRAWINGS

The invention is herein described, by way of example only, with reference to the accompanying drawings, wherein:

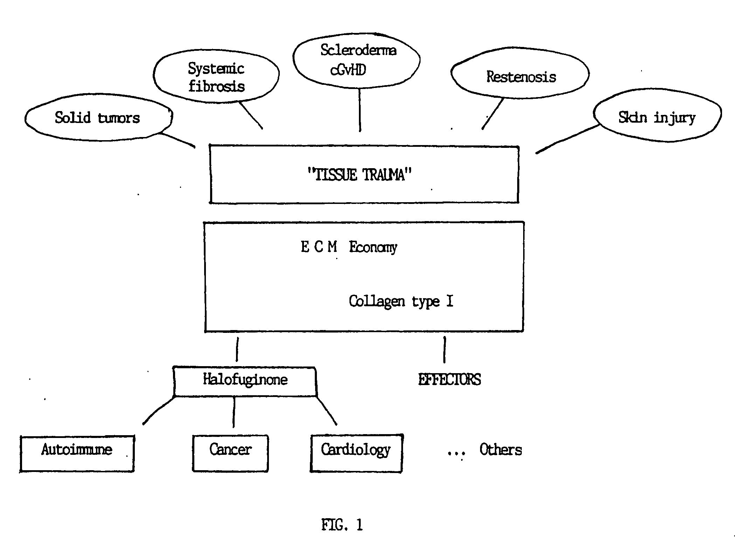

FIG. 1 illustrates certain exemplary aspects of the extracellular matrix economy;

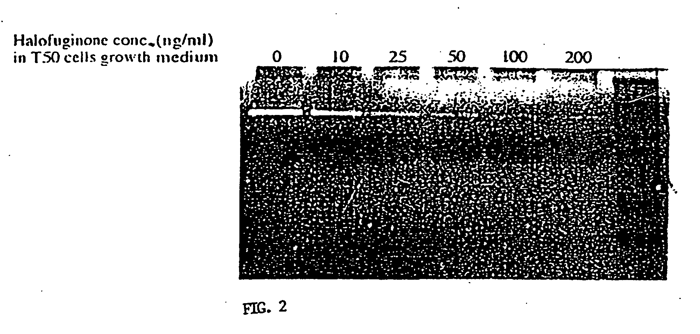

FIG. 2 illustrates the dose-dependent inhibition of type IV collagenase activity in T50 bladder carcinoma cell cultures in the presence of Halofuginone;

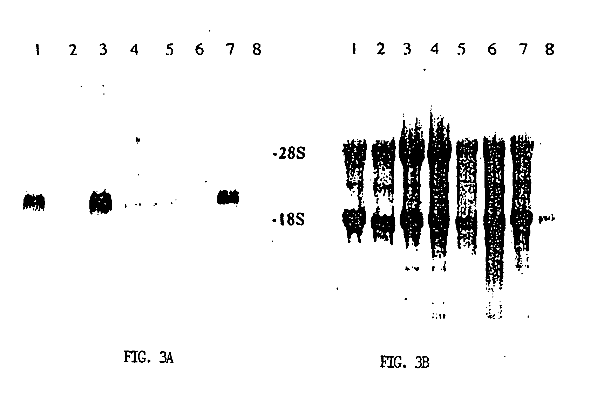

FIG. 3 illustrates the inhibition of the expression of the H19 gene in the RT112 and 5376 bladder carcinoma cell lines by Halofuginone;

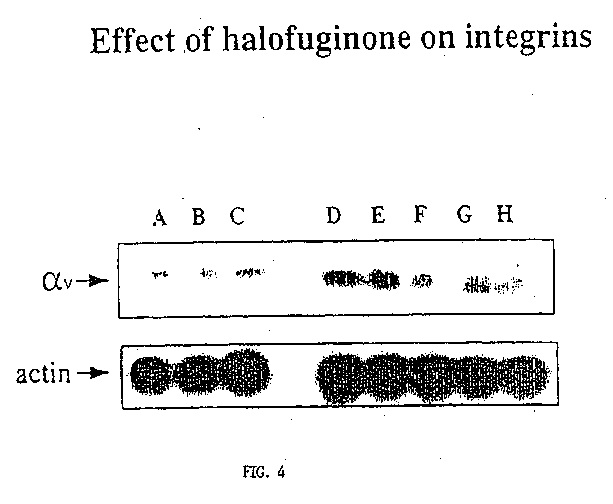

FIG. 4 shows a Northern blot with the effect of Halofuginone on Integrin αᵥ chain expression;

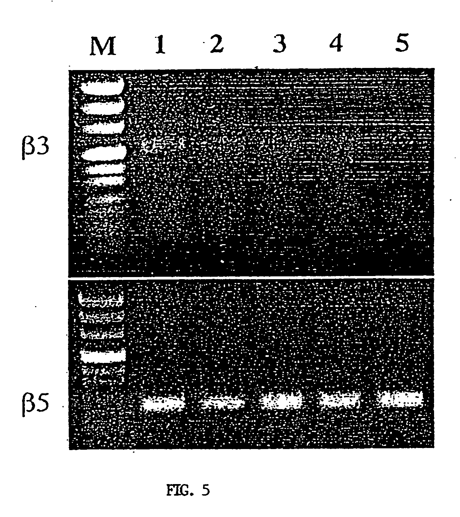

FIG. 5 shows the effect of Halofuginone on β subunit expression as determined by RT-PCR;

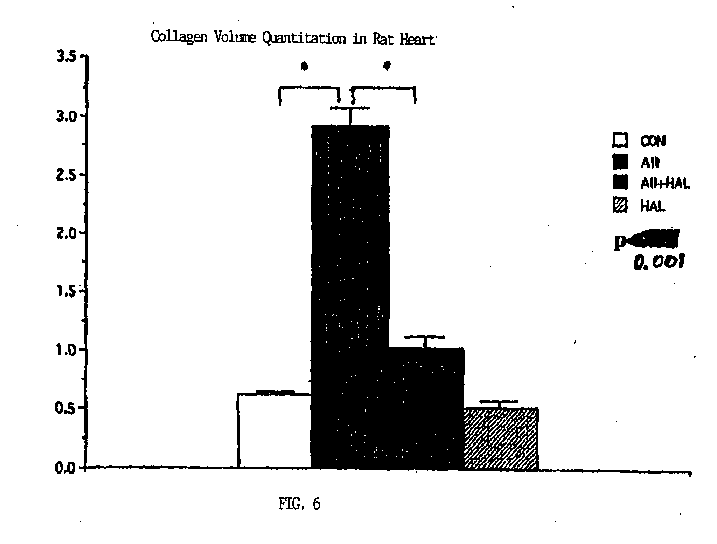

FIG. 6 illustrates the effect of Halofuginone on ventricular collagen volume fraction (CVF) in rat heart;

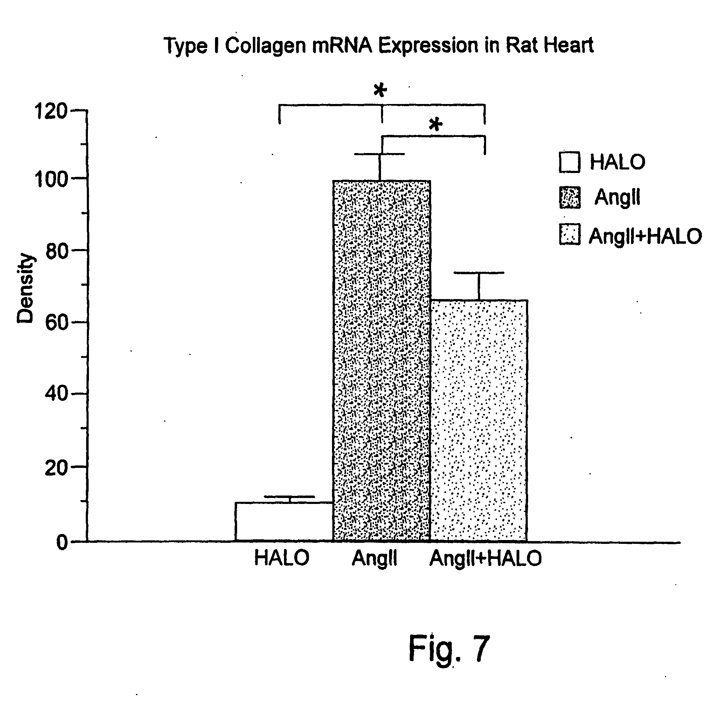

FIG. 7 illustrates the effect of Halofuginone on collagen α1(I) gene expression in rat heart; and

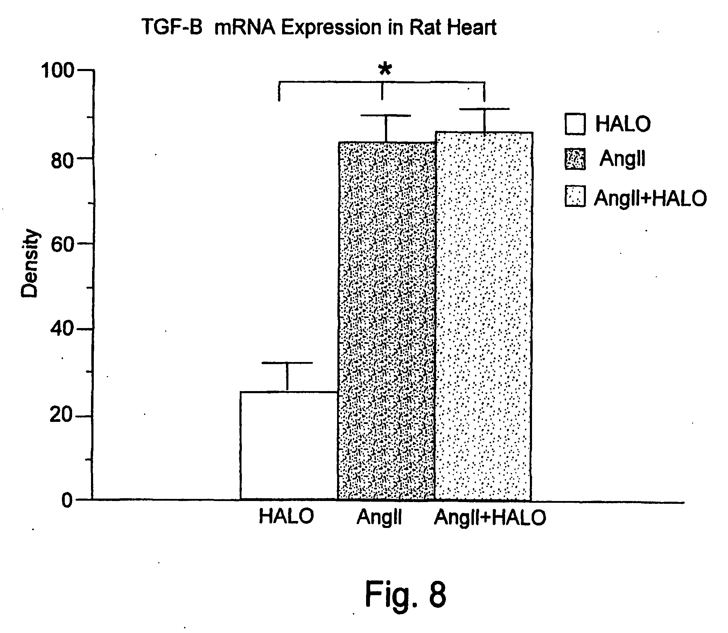

FIG. 8 illustrates the effect of Halofuginone on TGF-β expression in rat heart.

### BRIEF DESCRIPTION OF THE INVENTION

Unexpectedly, it has been found, as described in the examples below, that the underlying mechanism of action of Halofuginone in the inhibition of all of these pathogenic responses to tissue trauma involves the regulation of the extracellular matrix economy at the molecular level.

As disclosed herein the present experimental results also show that Halofuginone clearly inhibits the expression of NF-κB (nuclear factor κB), also in parallel with the inhibition of the expression of collagen. NF-κB has become the focus of intense interest, as it has been shown to become activated in cells through a wide variety of stimulating factors which are associated with stress or injury. NF-κB has been shown to be induced through a large number of factors, such as IL-1β (interleukin-1β) and tumor necrosis factor α (TNFα) [Mercurio and Manning; *Curr*. *Op. in Cell Biol*., **11**:226-232, 1999]. Interestingly, these specific factors have been shown in the present experimental results also to be inhibited by Halofuginone. Many inflammatory factors have also been shown to induce NF-κB, such that a wide variety of induction mechanisms are considered to converge on this particular target. Thus, the effect of Halofuginone on the inhibition of NF-κB expression may indicate at least one feature of the mechanism for inducing the effect of Halofuginone on the inhibition of fibrotic processes, while regulating and maintaining the physiologically normal and desirable extracellular matrix economy.

In turn, NF-κB has been shown to play an important role in the regulation of the expression of the type VII collagen gene (COL7A1). NF-κB mediates the effect of TNF-α on the expression of COL7A1, by binding to a particular portion of the promoter of this gene which is known as a "TNF-α response element" [*Kon et al., Oncogene,* **18**:1837-1844, 1999]. Interestingly, COL7A1 has two separate elements in the promoter region: the previously mentioned "TNF-α response element"; and a separate response element for TGF-β. Thus, the apparent effects of Halofuginone with regard to TNF-α and NF-κB, but not TGF-β, may ultimately enable Halofuginone to have the desired inhibitory effects on the pathological processes of fibrosis, substantially without detrimentally inhibiting or downregulating the physiologically normal and desirable extracellular matrix economy.

Another important molecular target of Halofuginone is the *cKrox* transcription factor for the expression of the collagen α1(I) gene. *cKrox* is a novel zinc finger-containing transcription factor which binds to the α1(I) and α2(I) collagen gene promoters, and was shown to repress transcription of the α1(I) procollagen promoter (Widom R L; Culic I; Lee J Y; Kom J H; *GENE,* (1997 Oct 1) **198**:407-20). As shown in the examples below, Halofuginone, in turn, was shown to potentiate the effect of *cKrox* and thus to potentiate inhibition of collagen synthesis.

*c-Krox* was also found to be present at much higher levels in skin than bone, which may explain why Halofuginone was able to decrease excessive collagen deposition in skin while not increasing the fragility of bone (Galera P; Musso M; Ducy P; Karsenty G; *PNAS,* (1994) **91**: 9372-6).

Other important effects which have been shown to occur through the administration of Halofuginone include decreasing the expression of the specific collagen chaperone heat shock protein HSP47 gene in parallel to the inhibition of the expression of the collagen α1(I) gene and decreasing the release of the cytokines IL-1β and TNFα, while inhibiting the expression of NF-κB; but without affecting the expression of TGFβ.

HSP47 has been shown to be a molecular chaperone which is specific for collagen, and in particular for procollagen [Nagata and Hosokawa; *Cell Structure and Function;* **21**:425-430, 1996]. HSP47 is a protein which resides in the endoplasmic reticulum (ER), and which binds to newly synthesized procollagen until the procollagen chain enters the Golgi body. HSP47 may assist in the formation of proper collagen protein structures, such as the triple helix structure of collagen. In further support of the functional relevance of HSP47 to collagen, previous results have shown that the expression of HSP47 in various cells is closely related to the expression of collagen [Nagata and Hosokawa; *Cell Structure and Function;* **21**:425-430, 1996]. In particular, increased expression of HSP47 was found during the progression of liver fibrosis which was induced by the administration of carbon tetrachloride to rats, also in parallel to the increased expression of collagen type I.

Interestingly, the present experimental results show that Halofuginone clearly inhibits the expression of HSP47 in parallel with the expression of collagen. These results support the hypothesis that Halofuginone acts at a common, root mechanism for a number of different processes related to the pathological synthesis and deposition of collagen and other ECM components within the fibrotic disease state.

In any case, all of these mechanisms are related to the regulation of the extracellular matrix economy. Such regulation is not merely inhibition of all processes related to the extracellular matrix and collagen deposition, since Halofuginone was previously shown by the inventors to inhibit excessive collagen deposition associated with keloids and other abnormal scar formation, yet Halofuginone did not decrease wound strength, as shown in U.S. Patent 5,852,024.

The proper regulation of the extracellular matrix economy leads to the inhibition of the pathological response to tissue trauma, such that all of the potential targets for the mechanism of action of Halofuginone and other effectors are able to prevent such pathological responses substantially without inhibiting or altering other desirable physiological activity. Indeed, the term "effector" is used herein to refer to substantially any compound or combination thereof which is capable of regulating the extracellular matrix economy, thereby specifically inhibiting pathological processes related to tissue trauma and mechanistically related processes.

The term "mechanistically related processes" includes those pathological conditions which share at least one underlying mechanism with abnormal responses to tissue trauma. For example, the metastasis of malignant cells is an example of a mechanistically related process, because such metastasis depends upon neo-angiogenesis, which in turn depends upon the deposition of extracellular matrix components including collagen, as shown in PCT Application No. WO 98/34613, filed on February 11, 1998.
Similarly, abnormal responses to tissue trauma such as adhesion formation also depend upon collagen deposition. Thus, all of these pathological processes can be said to be mechanistically related.

Other mechanisms of the "extracellular matrix economy" include, but are not limited to, the inhibition of angiogenesis, the prevention of ECM deposition, the inhibition of collagenase type IV production, the inhibition of integrin expression, the induction of apoptosis and the inhibition of H19 gene expression. The overall structure of the extracellular matrix economy, and the effect of Halofuginone in relation to this economy, is shown in Figure 1. As shown, the specific effects of Halofuginone on the extracellular matrix economy include the inhibition or amelioration of conditions associated with tumors, fibrosis, including renal fibrosis, scleroderma and GVHD, restenosis and skin injury. By mediating certain effects or factors associated with the extracellular matrix economy, Halofuginone and other effectors are able to inhibit collagen type I synthesis, hence ameliorating pathological conditions associated with tissue trauma while permitting normal, desirable physiological processes to continue.

With regard to specific aspects of these mechanisms, angiogenesis and ECM deposition have been previously described. Collagenase type IV is a pivotal metalloprotease enzyme involved in metastasis and cell invasion. Apoptosis is programmed cell death which, as noted previously, is blocked in malignant cells, which are therefore also described as "immortal". The H19 gene is a tumor-marker gene associated with the early stages of bladder carcinoma. More specifically, the H19 gene is a developmentally regulated gene whose expression peaks during fetal development when tissue differentiation is occurring. Chromosomal abnormalities within the region containing H19 are associated with early stages of malignancies such as Wilms' tumor, adrenocortical carcinoma, hepatoblastoma, rhabdomyosarcoma, lung tumors, trophoblastic tumors and bladder carcinoma [B. Tycko, *Am. J. Path.,* Vol. 144, p. 431-439, 1994; de Groot, N. *et al*., *Trophoblast Res.,* Vol. 8, p. 2285-2302, 1994; Rachmilewitz, J. *et al*., *Oncogene,* Vol. 11, p. 863-870, 1995].

The importance of the extracellular matrix economy is that the mechanism of action of Halofuginone is able to promote many desirable activities, such as the cytostatic inhibition of malignancies, the reduction or prevention of fibrosis and adhesions, and other pathological responses to tissue trauma, or mechanistically related activities thereof, substantially without causing undesirable side effects such as decreasing the strength of healed wounds or altering the remodeling of the structure of bone.

### DESCRIPTION OF PREFERRED EMBODIMENTS

Unexpectedly, it has been found, as described in the examples below, that the underlying mechanism of action of Halofuginone in the inhibition of all of these pathogenic responses to tissue trauma involves the regulation of the extracellular matrix economy at the molecular level. Such regulation includes the following effects: enhancing the activities of the *cKrox* transcription factor, which in turn represses transcription of the α1(I) procollagen promoter; decreasing the expression of the collagen molecular chaperone, HSP47, in parallel to the inhibition of the expression of the collagen α1(I) gene; decreasing the release of the cytokines IL-1β and TNFα, while inhibiting transcription of NF-κB; but without affecting the expression of TGFβ.

Furthermore, Halofuginone is also involved in other aspects of the extracellular matrix economy, including the inhibition of collagenase type IV production and the inhibition of H19 gene expression, as well as the overall regulation of ECM (extracellular matrix) deposition and remodeling, the amelioration and/or prevention of chronic inflammatory disease, and the inhibition of neo-angiogenesis. Other mechanisms of the "extracellular matrix economy" include, but are not limited to, the inhibition of angiogenesis, the prevention of ECM deposition, the inhibition of collagenase type IV production, the inhibition of integrin expression, the induction of apoptosis and the inhibition of H19 gene expression. Such specific regulation of the extracellular matrix economy has never been demonstrated before, particularly *in vivo*.

While the invention will now be described in connection with certain preferred embodiments in the following figures and examples so that aspects thereof may be more fully understood and appreciated, the invention is not intended to be limited to these particular embodiments. On the contrary, all alternatives, modifications and equivalents are included as within the scope of the invention as defined by the appended claims. Thus, the following figures and examples which include preferred embodiments will serve to illustrate the practice of this invention, it being understood that the particulars shown are by way of example and for purposes of illustrative discussion of preferred embodiments of the present invention only, and are presented in the cause of providing what is believed to be the most useful and readily understood description of formulation procedures as well as of the principles and conceptual aspects of the invention.

The present invention may be more readily understood with reference to the following illustrative examples and figures. It should be noted that although reference is made exclusively to Halofuginone, it is believed that the other quinazolinone derivatives described and claimed in U.S. Patent 3,320,124 have similar properties.

### Example 1

### Promotion of cKrox Activity and Inhibition of Collagen Type I

### Gene Expression by Halofuginone

As noted previously, one of the most important targets for the action of Halofuginone and other related quinazolinones and effectors is the promotion of *cKrox* activity and the concomitant inhibition of collagen type I gene expression. These two activities were demonstrated with Halofuginone as follows.

First, the ability of Halofuginone to inhibit collagen type I gene expression was demonstrated as follows. Myometrial and leiomyosarcoma cells were taken from the same patient and were plated into 10 cm plates in DMEM supplemented with 10% FCS. When the cells reached 80% confluence, the medium was replaced by serum free DMEM plus 0.1% BSA for 48 hours, washed and exposed to increasing concentrations of Halofuginone in the same medium for about 48 hours at about 37 °C. The cells were then harvested and subjected to RNA extraction and Northern blot analysis for collagen type I gene expression. Halofuginone inhibited collagen type I gene expression (products at 5.4 and 4.8 kb) in a dose-dependent manner.

Next, human skin fibroblasts were taken from a subject and were maintained in primary culture. Control cells were treated with vehicle while Halofuginone-treated cells were treated with Halofuginone as previously described. The cells were then harvested and subjected to RNA extraction and Northern blot analysis for collagen type I gene expression and for *cKrox* gene expression. Halofuginone promoted *cKrox* gene expression while simultaneously inhibiting collagen type I gene expression. Thus, one important molecular target for the mechanism of action of Halofuginone is clearly the enhancement of *cKrox* gene expression and hence *cKrox* activity, which in turn leads to the inhibition of collagen type I gene expression.

Such targeting of the action of Halofuginone is novel, and has not been taught nor suggested by the background art. However, clearly the elucidation of this mechanism is important for the development and design of new treatments for the pathological processes associated with tissue trauma. Furthermore, such results provide a clear mechanistic explanation for the ability of Halofuginone to inhibit pathological collagen synthesis, while enabling normal physiological processes associated with collagen to proceed without unwanted side effects. In particular, molecules and chemical compositions which also are able to inhibit pathological collagen synthesis, while enabling normal physiological processes associated with collagen to proceed without unwanted side effects, are now possible by targeting the potentiation of *cKrox* gene expression and/or activity for therapeutic intervention.

### Example 2

### Inhibition of Type IV Collagenase Production by Halofuginone in vitro

Another important feature of the regulation of the extracellular matrix economy is the inhibition of type IV collagenase production by Halofuginone.

Tumor cells secrete enzymes which digest the ECM, enabling the cells to burrow through neighboring tissue and to invade other tissues. Numerous studies have linked matrix metalloproteases (MMP), especially type IV collagenase, to the process of tumor invasion and metastasis. Type IV collagenase appears as two 72 and 92 kDa proteins encoded by a unique mRNA.

As demonstrated in Figure 2, a profound inhibition of the activity of MMP2 (72 kDa type IV collagenase) in T50 bladder carcinoma cell cultures was exerted in the presence of 25 ng/ml Halofuginone, while an almost complete inhibition was obtained at 100 ng/ml Halofuginone. Sub-confluent cell cultures were incubated for 6 - 24 h in serum-free DMEM. The collagenolytic activity was determined on a gelatin impregnated (1 mg/ml. Difco, Detroit, MI) SDS-PAGE 8% gel. Briefly, culture media samples were separated on the substrate impregnated gels under non reducing conditions, followed by 30 min incubation in 2.5% Triton X-100 (BDH, England). The gels were then incubated for 16 h at 37°C in 50 mM Tris, 0.2 M NaCl, 5 mM CaCl₂, 0.02% Brij 35 (weight/volume) at pH 7.5. At the end of incubation period, the gels were stained with 0.5% Coomassie G250 (Bio-Rad Richmond CA) in methanol/acetic acid/H₂O (30:10:60). The intensity of the various bands was determined on a computerized densitometer (Molecular Dynamics type 300A).

Halofuginone was also found to inhibit cell invasion through matrigel ECM, using the Boyden chamber invasion assay (data not shown). Such inhibition supports the inclusion of type IV collagenase inhibition as part of the extracellular matrix economy mechanism, in which Halofuginone inhibits undesirable pathological processes such as tumor growth, progression and metastasis, as described previously.

### Example 3

### Inhibition of Tumor-Marker Gene Expression by Halofuginone in vitro

Yet another aspect of the extracellular matrix economy is the regulation of tumor-marker gene expression, and in particular of the inhibition of the expression of the H19 gene.

The H19 gene is a developmentally regulated gene whose expression peaks during fetal development when tissue differentiation is occurring. The H19 gene is parenterally imprinted, expressed only by the maternal allele. H 19 is also a tumor-marker gene, associated with early stages of malignancies such as Wilms' tumor, adrenocortical carcinoma, hepatoblastoma, rhabdomyosarcoma, lung tumors, trophoblastic tumors and bladder carcinoma. The experimental method was as follows.

RT112 and 5376 human bladder carcinoma cell lines were cultured in the absence and presence of Halofuginone (130 ng/ml, added 24 h or 72 h after seeding), and the expression of the H19 gene was evaluated by Northern blot analysis (Nagler, A. *et al., Arterioscler. Thromb. Vasc. Biol*., Vol. 17, p. 194-202, 1997). Exposure to Halofuginone resulted in a substantial reduction in the expression of the H19 gene in the RT112 and 5376 bladder carcinoma cell lines which were tested, as shown in Figure 3. Such inhibition also supports the inclusion of the inhibition of H19 gene expression as part of the extracellular matrix economy mechanism, as described previously.

### Example 4

### Inhibition of Integrin Expression

Another important aspect of the effect of Halofuginone and related quinazolinones on the extracellular matrix economy involves the ability of these compounds to inhibit integrin expression, as exemplified by the effect of the illustrative compound Halofuginone.

Integrins have been shown to function *in vivo* in vasculogenesis and angiogenesis. Injection of neutralizing antibody against the β1 subunit blocked the formation of an aortic lumen in quail embryos (Drake, C.J. *et al*., *in vivo. Dev. Dyn*. Vol. 193, p. 83-91, 1992). Cheresh and colleagues have provided evidence that αᵥβ₃ is required for blood vessel growth (Brooks, P.C. *et al*., *Science* Vo. 264, p. 569-571, 1994). An antibody (LM609) against the αᵥβ₃ integrin complex inhibited normal vessel growth and also FGF-2 stimulated or tumor-induced angiogenesis in the CAM assay, but did not disrupt preexisting vessels. The mechanism by which anti-αᵥβ₃ mAb disrupts angiogenesis appears to involve apoptosis. A single intravascular injection of a cyclic RGD peptide antagonist of αᵥβ₃ integrin or of the LM609 monoclonal antibody leads to the rapid regression of human tumors transplanted into the CAM. Tumor cells that fail to express the αᵥ gene and hence the αᵥβ₃ integrin, lose their adhesion capability and exhibit a significantly reduced tumorigenicity upon transplantation into athymic nude mice. Stable transfection of the αᵥ cDNA to these cells resulted in the full restoration of their tumorigenic potential (Felding-Habermann, B. *et al, J. Clin. Invest.,* Vol. 89, p. 2018-2022, 1992). Furthermore, Halofuginone has been found to inhibit angiogenesis and tumor growth.

Therefore, the effect of Halofuginone was investigated on the expression of αᵥ, β₃ and β₅ integrin subunits in the highly aggressive MDA 435 human breast carcinoma cell line. Cells were cultured in the absence (Figure 4, lanes A & B) or presence of increasing concentrations (10-400 ng/ml) of Halofuginone for 24 h (lane C: 400 ng/ml), 48 h (lanes D-G: 10, 50, 200, and 400 ng/ml, respectively) or 72 h (Figure 4, lane H: 400 ng/ml). Total RNA was extracted, subjected to 1.1 % formaldehyde-agarose gel electrophoresis, transferred to a nylon membrane and hybridized with ³²P-labeled PCR probe corresponding to αᵥ. As demonstrated in Figure 4, exposure of the breast carcinoma cells for 48 h to 10 and 50 ng/ml Halofuginone resulted in up regulation of the αᵥ mRNA (Figure 4, lanes D & E). This effect was minimal at higher concentrations (200-400 ng/ml) of Halofuginone (Figure 4, lanes F-H). Next, RT-PCR was used to analyze the effect of Halofuginone on expression of the β₃ and β₅ integrin chains by the MDA 435 breast carcinoma cells. As shown in Figure 5, Halofuginone inhibited the expression of the mRNA in a dose-dependent manner, yielding an almost complete inhibition at 200 ng/ml (Figure 5, lane 1: control; lanes 2-5: 24 h exposure to 10, 50, 200 and 400 ng/ml Halofuginone, respectively). In contrast, there was no effect on expression of the β₅ mRNA. As the αᵥβ₃ integrin complex plays an important role in tumor angiogenesis, the anti-angiogenic effect of Halofuginone may be mediated in part by its inhibition of the β₃ gene expression.

Thus, the inhibition of the expression of integrin genes is clearly another aspect of the regulation of the extracellular matrix economy by Halofuginone.

### Example 5

### Inhibition of Cardiac Fibrosis

As noted previously, the ability of certain molecules to regulate the extracellular matrix economy by inhibiting abnormal responses to tissue trauma and other mechanistically related pathological processes, while maintaining normal physiological processes, has been exemplified by the effect of the illustrative compound Halofuginone. However, none of these results taught or suggested the suitability of quinazolinone-containing compounds such as Halofuginone as a treatment for cardiac fibrosis. Such a result is unexpected because cardiac tissue is composed of highly differentiated cells which must maintain a high overall level of organization in order to function effectively.

Furthermore, myocardial tissue must contract as a single unit in response to an electrical signal, which is not a property associated with other, previously studied tissues for the treatment of fibrosis and other types of tissue traumas with Halofuginone. This property is specific to cardiac tissues, and increases the damaging effect of fibrosis, since fibrotic tissue cannot contract in this manner. Second, damaged myocardial tissue contracts improperly: rather than initiating the contraction of the heart at a single focal point, followed by the sweep of the potential throughout the heart tissue, arrhythmias can develop in damaged tissues in which many such focal points arise, causing improper contractions and eventually death. Third, the tissue of the heart must function as a single unit. Other tissues, such as lung and liver, are composed of different tissue types and structures which can more or less function independently. However, the entire heart must function as a single unit. Thus, the preservation restoration of proper myocardial function by Halofuginone, either before or after the fibrotic process has begun, cannot be predicted or taught from the prior art.

Furthermore, Halofuginone has only been shown to be a collagen type I inhibitor. However, the formation of fibrotic tissue in the heart is characterized by the deposition of abnormally large amounts of extracellular matrix components. Thus, the ability of Halofuginone to inhibit collagen type I synthesis and deposition cannot predict the ability of Halofuginone to slow, reduce or other ameliorate the pathogenesis of cardiac fibrosis.

Furthermore, as demonstrated below, Halofuginone is able to prevent cardiac fibrosis by inhibiting the deposition of type I collagen, without downregulating or otherwise altering the synthesis of TGF β (transforming growth factor), which is a cytokine generally affecting the synthesis and deposition of several ECM components. Without wishing to be limited by a single mechanism, Halofuginone may be exerting its effect through an influence on type I collagen transcription. Thus, the effects of Halofuginone are specific and restricted, yet are able to prevent cardiac fibrosis.

Although the pathogenesis of cardiac fibrosis is not fully understood, animal models for the disease have been successfully developed. Cardiac fibrosis has been induced in rats by the chronic administration of angiotensin II (Ang II).

Compounds which are intended for the inhibition of cardiac fibrosis must be tested in an *in vivo* model, such as the Ang II model described above, for their ability to slow or halt the pathological process leading to deposition of fibrotic tissue. Such experiments were conducted for the collagen type I synthesis inhibitor Halofuginone, as described in greater detail below.

Histological examination of heart samples from control and Ang II (angiotensin II)-treated rats revealed that Ang II induced specific morphological changes in rat heart, including increased collagen fiber content. Halofuginone substantially inhibited the occurrence of these morphological changes, resulting in rat heart of more normal appearance.

The experimental method was as follows. Male Sprague-Dawley rats were divided into four groups. Two groups were chronically infused with Ang II at the rate of 0.150 ng/min by implanted mini-pump. This dosage regimen will induce severe cardiac fibrosis. The other two groups of rats, control rats, were injected with saline. One group of Ang II-treated rats and one control group were daily injected intraperitoneally with 16 micrograms of Halofuginone. At the end of the experimental period, the rats were sacrificed and the heart was removed and weighed.

Heart samples were taken for histological examination. Briefly, the tissue samples were collected into phosphate-buffered saline (PBS) and fixed overnight in 4% paraformaldehyde in PBS at 4 °C. Serial 5 µm sections were prepared after the samples had been dehydrated in graded ethanol solutions, cleared in chloroform and embedded in Paraplast. Differential staining of collagenous and non-collagenous proteins was performed with 0.1 % Sirius red and 0.1 % fast green as a counter-stain in picric acid. This procedure stains collagen red [Gascon-Barre, M., *et al., J. Histochem. Cytochem*., **37**:377-381, 1989].

Heart samples were then hybridized with a probe for rat collagen α1(I) expression, or with a probe for TGF β1 expression. For hybridization with one of the genetic probes, the sections were deparafinized in xylene, rehydrated through a graded series of ethanol solutions, rinsed in distilled water for 5 minutes and then incubated in 2X SSC at 70 °C for 30 minutes. The sections were then rinsed in distilled water and treated with pronase, 0.125 mg/ml in 50 mM Tris-HCl, 5 mM EDTA, pH 7.5, for 10 minutes. After digestion, the slides were rinsed with distilled water, post-fixed in 10% formalin in PBS and blocked in 0.2% glycine. After blocking, the slides were rinsed in distilled water, rapidly dehydrated through graded ethanol solutions and air-dried for several hours. Before hybridization, the 1600 bp rat collagen α1(I) insert was cut out from the original plasmid, pUC18, and inserted into the pSafyre plasmid. The sections were then hybridized with this probe after digoxigenin-labeling [M. Pines *et al*., *Matrix Biology,* **14**:765-71, 1996]. Similarly, other slides were hybridized with a probe for TGF-β1.

Figure 6 shows the result of collagen volume quantitation of rat heart after videodensitometry. Sections of rat liver tissue were stained with Sirius red to demonstrate collagen content of the tissue. A low volume of collagen was observed in control rats (CON) and rats which received Halofuginone alone (HAL). A high volume of collagen was observed in rats which received Ang II (A II) alone, but was markedly reduced in rats given both Ang II and Halofuginone (A II + HAL), indicating the ability of Halofuginone to substantially inhibit the pathophysiological process of fibrosis induced by Ang II. Indeed, the ventricular collagen volume fraction (CVF) was increased by threefold (p<0.05) in rats treated with Ang II, compared to rats treated with either Halofuginone alone or Halofuginone plus Ang II. In addition, the ventricular CVF for control rats was identical to that for rats treated with Halofuginone alone. Indeed, over a two week period, Halofuginone did not alter the ventricular CVF when administered alone, without Ang II. Thus, Halofuginone had a strong ability to prevent the increase in ventricular CVF which is induced by Ang II, without having effect alone on ventricular CVF.

Figure 7 shows the results of densitometery measurements after *in situ* hybridization of a section of rat heart tissue with rat collagen α1(I) probe. A low expression of collagen α1(I) gene is seen in heart tissue of rats given Halofuginone alone (HALO). A marked increase in the expression of collagen α1(I) gene was seen in the liver of rats given Ang II alone (Ang II). Rats given both Halofuginone and Ang II show a marked reduction in the expression of collagen α1(I) gene (Ang II + HALO), as compared to rats given Ang II alone. Although this dose of Halofuginone substantially reduced the increase in rat collagen α1(I) gene expression caused by Ang II, it did not completely inhibit such expression. However, the substantially reduced rat collagen α1(I) gene expression indicates that Halofuginone is effective against the pathological induction of expression by Ang II. Thus, the five fold rise in type I collagen mRNA level (p<0.05), induced by Ang II, was attenuated by Halofuginone.

Figure 8 shows that although Ang II caused an increase in the expression of mRNA for TGF P (p<0.05), this enhancement was not affected by the administration of Halofuginone. In particular, the level of TGF β expression was significantly and equally higher in rats given either Ang II (AngII) or Ang II plus Halofuginone (Ang II + HALO), than in rats given Halofuginone alone (HALO). Thus, Halofuginone does not appear to inhibit the processes of extracellular matrix synthesis and deposition which are controlled by TGF β.

Thus, Halofuginone is able to prevent cardiac fibrosis by inhibiting the deposition of type I collagen, without altering the synthesis of TGF β, which is a cytokine affecting the synthesis and deposition of several ECM components. The effects of Halofuginone appear to be specific to a particular aspect of the pathway for extracellular matrix synthesis, which is neither taught nor suggested by the background art. Therefore, the specificity of Halofuginone appears to be directed to a particular mechanism of collagen synthesis and deposition, a mechanism which does not appear to be controlled by TGF β.

Overall, Halofuginone was able to prevent the appearance of the effects of Ang II-induced fibrosis on all levels, including marked reduction of gross and fine morphological changes caused by Ang II-induced fibrosis. Clearly, the effects of Halofuginone are both potent and specific for the prevention of the morphological changes produced during the pathological process of cardiac fibrosis.

### Example 6

### Co-regulation of Multiple Genes with Halofuginone

As described in greater detail below, unexpectedly Halofuginone has been shown to be effective for the co-regulation of multiple genes whose activity is not known to be co-regulated. Such co-regulation is specific, in that certain genes which are known to be potentially linked to the regulated genes in other systems are not co-regulated by Halofuginone. Furthermore, such specific co-regulation clearly indicates a common, underlying mechanism for all of these effects of quinazolinone derivatives such as Halofuginone.

In particular, experimental results show that Halofuginone causes a number of effects which were not previously known to be necessarily related and which include the decrease in the expression of collagen type I, in particular by inhibiting the expression of the collagen α1(I) gene; inhibiting collagenase type IV production; inhibiting H19 gene expression; decreasing the expression of the HSP47 gene in parallel to the inhibition of the expression of the collagen α1(I) gene; decreasing the release of the cytokines IL-1β and TNFα, while inhibiting the expression of NF-κB; but without affecting the expression of TGFβ.

As previously described in greater detail, Halofuginone appears to exert a number of related inhibitory effects with regard to the expression of these genes. For example, Halofuginone has been shown to downregulate the expression of HSP47, a molecular chaperone which is specific for collagen, in parallel with the expression of type I collagen. On the other hand, increased expression of HSP47 was found during the progression of liver fibrosis which was induced by the administration of carbon tetrachloride to rats, also in parallel to the increased expression of collagen type I, such that the parallel inhibition of both HSP47 and collagen type I by Halofuginone may indicate one aspect of the mechanism according to which Halofuginone affects a number of different processes related to the pathological synthesis and deposition of collagen and other ECM components within the fibrotic disease state.

Furthermore, Halofuginone was clearly shown below to inhibit the expression of NF-κB (nuclear factor κB), also in parallel with the inhibition of the expression of HSP47 and with the expression of collagen. NF-κB was shown to be induced through a large number of factors, such as IL-1β (interleukin-1β) and tumor necrosis factor α (TNFα) [Mercurio and Manning; *Curr. Op. in Cell Biol*., **11**:226-232, 1999], which were among the specific factors which were also be inhibited by Halofuginone in the present experiments.

Thus, the apparent effects of Halofuginone with regard to TNF-α and NF-κB, but not TGF-β, may ultimately enable Halofuginone to have the desired inhibitory effects on the pathological processes of fibrosis, substantially without detrimentally inhibiting or downregulating the physiologically normal and desirable extracellular matrix economy.

### Inhibition of Biochemical Processes

The ability of Halofuginone to inhibit various biochemical processes according to a set of assays for determining the percent inhibition of specific binding or activity of particular cellular proteins is described in greater detail in Table 1 below: The assays were performed by MDS Panlabs Inc. (Bothell, Wisconsin, USA). These assays included: the inhibition of release of the cytokines IL-1β and TNF-α; and the inhibition of the transcription of NF-κB. The results were as follows:

**Table 1**

| Primary Assay | IC₅₀ |
|---|---|
| cytokine IL-1β release | 31.7 nM |
| cytokine TNF-α release | 6.8 nM |
| NF-κB transcription | 25.0 nM |

Thus, these results clearly show the constellation of inhibitory effects by Halofuginone as previously described. Furthermore, these results show a strong and significant effect by Halofuginone for these specific functions, while other assayed functions remained substantially unaffected by Halofuginone (data not shown).

### Investigation of Changes in Gene Expression with Halofuginone

As described in greater detail below, the effect of Halofuginone on the expression of multiple genes was investigated by using the Atlas cDNA Expression Arrays (Clontech Ltd.), which enable a large number of genes to be investigated simultaneously. The results are shown in Table 2 (down-regulation of genes after treatment with Halofuginone) for Atlas functional arrays; and in Tables 3 (up-regulation of genes after treatment with Halofuginone) and 4 (down-regulation of genes after treatment with Halofuginone) for Atlas human interaction arrays. The experimental method was as follows.

The Atlas Human cDNA Expression Array, cat. No. 7740-1, was used. The Atlas cDNA Expression Array includes 588 human cDNAs spotted in duplicates on a positive charged nylon membrane, nine housekeeping control cDNAs for normalizing mRNA abundance, along with plasmid and bacteriophage DNAs which are included as negative controls to confirm hybridization specificity. The cDNAs are represent and arrayed into several different functional classes, including transcription factors, cytokines, oncogenes, and tumor suppressor genes. The cDNAs immobilized on each Atlas Array have been specially prepared to minimize the problem of nonspecific hybridization. Each cDNA fragment is 200 to 600 bp and has been amplified from a region of the transcript that lacks the poly-A tail, repetitive elements, or other highly homologous sequences. A transparent Orientation Grid was also used to allow identification of cDNAs corresponding to positive hybridization signals.

First, 1 µg of each RNA population was reverse-transcribed by using the reagents provided and [α-³²P]dATP. The radioactively labeled, complex cDNA probes were separately hybridized overnight to the Atlas Arrays using ExpressHyb Hybridization Solution, which was also provided. After a high-stringency wash, the hybridization pattern was analyzed by autoradiography and/or quantified by phosphorimaging, as described in greater detail below. The relative expression levels of a given cDNA in two different RNA sources was assessed by comparing the signal obtained with a probe from one RNA source to that obtained with a probe from another source.

Total RNA was prepared from primary human fibroblasts. Cells were grown in 15 mm culture dishes in the presence of 10% FCS in DMEM (Dulbecco's Modified Eagle Medium) culture media. At about 70-90% confluency, the cells were treated with 10-⁸ M Halofuginone for 1hr. Both treated and untreated cells, were subjected to total RNA preparation using SV Total RNA Isolation System (Promega, cat. No. Z3100) or Tri Reagent (Molecular Research Center, Inc., cat No. TR-118).

According to the protocol of Promega, 2 ml lysis buffer was added to 15 mm plate, the lysed cells were collected using a 200-1000 microliter tip and transferred to the next plate until the cells in the whole 5 plates were lysed and collected. The lysate was transferred to microcentrifuge tubes, 175 µl of lysate per tube, total of 10 tubes per each 5 plates. 350µl of SV RNA Dilution Buffer was added to each 175 µl of cell lysate and the solution was mixed by inverting the tube 3-4 times. Following incubation at 70 °C for 3 min, the lysates were centrifuged at 11,000g for 10 min at R.T.

For each sample, a Spin Column Assembly was prepared. Each Spin Column Assembly consists of a Spin Basket and a Collection Tube. The caps on the Spin Baskets were removed and the Collection Tubes were labeled and placed in a microcentrifuge tube rack. The cleared lysate was transferred to fresh microcentrifuge tubes, without disturbing the debris. 200 µl of 95% ethanol was added to each lysate tube and mixed by pipetting 3-4 times. The mixture was transferred to the Spin Column Assembly and centrifuged at 11,000g for 1 min at R.T.

The Spin Basket was taken from the Spin Column Assembly and the liquid in the Collection Tube was discarded. The Spin Basket was returned to the collection Tube and 600µl of SV RNA Wash solution was added to the Spin Column Assembly and the columns were centrifuged at 11,000g for 1 min. The Collection Tube was emptied as before and set in a rack. For each isolation to be performed, DNase incubation mix was prepared by combining 40 µl Yellow Core Buffer, 5µl 0.90M MnCl₂ and 5µl of DNase I enzyme per sample in a sterile tube (in this order). The incubation mix was mixed gently by pipetting (not vortexing) and kept on ice. 50µl of this freshly prepared DNase incubation mix was applied directly to the membrane inside the Spin Basket, being sure that the solution was in contact with and thoroughly covering the membrane. Following incubation for 15 min at R.T., 200µl of SV DNase Stop Solution was added to the Spin Basket and centrifuged at 11,000g for 1 min. 600 µl of SV RNA Wash Solution was added followed by centrifugation at 11,000g for 1 min. The Collection Tube was emptied, 250µl of SV RNA Wash Solution was added and centrifuged at high 11,000g for two min.

For each sample, one Elution Tube was prepared; the Spin Basket was transferred from the Collection Tube to the Elution Tube, and 100 µl Nuclease-Free Water was added to the membrane, while completely covering the surface of the surface of the membrane with the water. The Spin Basket Assemblies were placed in the centrifuge with the lids of the Elution Tubes facing out and centrifuged at 11,000g for one min. The Spin Basket was removed and discarded.

According to Tri Reagent Procedure, cells were lysed directly in the culture dishes. 1 ml of Tri Reagent was added to each plate and the cell lysate was passed several times through a pipette. Each 5 plates (treated and untreated) were collected together. The homogenates were stored at R.T. for 5 min to permit the complete dissociation of nucleoprotein complexes. The homogenates were supplemented with 0.2 ml chloroform per each 1 ml of Tri Reagent and the samples were shaken vigorously for 15 seconds. The resulting mixture was incubated at R.T. for 15 min and centrifuged at 12,000g for 15 min at 40 °C. Following centrifugation, the mixture was separated into a lower red, phenol-chloroform phase, interphase, and the colorless upper aqueous phase containing the RNA. The aqueous phase was transferred to a fresh tube and RNA was precipitated by introducing of 0.5 ml of isopropanol per each 1ml of Tri Reagent used for the initial step.

Samples were incubated at R.T. for 10 min and centrifuged at 12,000g for 8 min at 40 °C. The supernatant was discarded and RNA pellet was washed with 75% ethanol (at least 1 ml per initially used Tri Reagent) by vortexing and subsequent centrifugation at 7,500g for 5 min at 40 °C. The resulted RNA was air dried and resuspended in water.

The yield of total RNA obtained was determined spectrophotometrically at 260 nm, where 1 absorbance unit (A260) equals 40µg of single-stranded RNA/ml. The integrity of the purified RNA was determined also by agarose gel electrophoresis in which the ratio of 28S to 18S eucaryotic ribosomal RNAs should be approximately 2:1 by ethidium bromide staining, indicating that no gross degradation of RNA has occurred.

Contamination by genomic DNA is particularly troublesome. Therefore, all RNA samples were treated with RNase-free DNase I prior to being used as a probe. The following reagents were combined for each sample:
500 µl Total RNA (0.5 µg)
100 µl 10x DNase I Buffer (400mM Tris-HCl pH 7.5; 100mM NaCl; 60mM MgCl₂)
50 µl DNase I (RQ1 RNase-Free DNase, Promega, cat No. M6101; undiluted; 1 unit/µl)
395 µl Deionized H₂O

The solution was mixed well by pipetting up and down several times. The reacting mixtures were incubated at 37 °C for 1 hr in a water bath followed by addition of 100 µl of 10X Termination Mix (0.1M EDTA [pH 8.0]; 1 ng/ml glycogen [type IX from bovine liver, Sigma, Catalog No. G0885]) and mixing by pipetting up and down.

Each reaction was split into two 1.5-ml microcentrifuge tubes (550 µl per tube) and 550 µl of phenol:chloroform:isoamylalcohol (25:24:1) was added to each tube, the mixtures were vortexed thoroughly and centrifuged in a microcentrifuge at 14,000 rpm for 10 min to separate phases. The top aqueous layer was carefully transferred to a fresh 1.5-ml microcentrifuge tube while the interface and lower phase were discarded. After repeating this extraction on the obtained aqueous phase, 550 µl of a mixture of chloroform:isoamylalcohol (24:1) was added to the final obtained aqueous layer, followed by vigorous vortexing. The tubes were centrifuged at 11,000g for 10 min to separate phases and the resulting top aqueous layer was removed and transferred to a 2.0-ml microcentrifuge tube. 1/5 volume (100µl) of 7.5 M NH₄OAc and 2.5 volumes (1.5 ml) of 96% ethanol were added, the mixtures were vortexed thoroughly and centrifuged at 14,000 rpm for 20 min. The supernatant was removed carefully and the pellet was gently overlaid with 100µl of 80% ethanol and centrifuged at 14,000 rpm for 10 min. After removing the supernatant, the pellet was dried for about 10 min to evaporate residual ethanol. The pellet was dissolved in 250 µl of deionized H₂O, the two identical tubes were combined and the RNA was subjected to oligo (dT) purification of poly A+ RNA.

Total RNA was subjected to poly A+ RNA isolation using Promega's PolyATract mRNA Isolation Systems III (cat No. Z5300). 500 µl volume of total RNA was incubated in a water bath at 65 °C for 10 min. 3 µl of the Biotinylated-Oligo (dT) Probe and 13 µl of 20x SSC were added to the RNA, mixed gently and incubated at R.T. until completely cooled (about 10 min). Streptavidin-Paramagnetic Particles (SA-PMPs) (tube per RNA sample) were resuspended by gently flicking the bottom of the tube until they were completely dispersed and then they were captured by placing the tube in the Magnetic Stand until the SA-PMPs have been collected at the side of the tube (approximately 30 seconds). The supernatant was removed and the SA-PMPs were washed three times with 0.5x SSC (0.3 ml per wash), each time they were captured using the Magnetic Stand and then the supernatant was removed carefully. The washed SA-PMPs were resuspended in 0.1 ml of 0.5x SSC.

The entire contents of the annealing reaction was added to the tube containing the washed SA-PMPs, the mixture was incubated at R.T. for 10 min (the mixture was gently mixed by inversion the tube every 1-2 min) and the SA-PMPs were captured using the Magnetic Stand and the supernatant was carefully removed without disturbing the SA-PMP pellet. The particles were washed four times with 0.1x SSC (0.3 ml per wash) by gently flicking the bottom of the tube until all of the particles were resuspended. After the final wash attention was paid to remove as much of the aqueous phase as possible without disturbing the SA-PMP particles. To elute the mRNA, the final SA-PMP pellet was resuspended in 0.1 ml of the RNase-Free Water and the particles were gently resuspended by gently flicking the tube. The SA-PMPs were magnetically captured and the eluted mRNA aqueous phase was transferred to a fresh tube.

The elution procedure was repeated by resuspending the SA-PMP pellet in 0.15 ml of RNase-Free Water, capturing the particles and pooling the eluted mRNA with the mRNA eluted in the previous step. In order to remove any particles, the eluted mRNA was centrifuged at 10,000g for 10 min and the mRNA was carefully transferred to a fresh tube.

The concentration and purity of the eluted mRNA was determined by spectrophotometry as described above.

In order to prepare cDNA probes, cDNA was synthesized from poly A+ RNA. 1 µg of Poly A+ RNA was subjected to a cDNA synthesis using Clontech Atlas cDNA Expression Array ingredients. A Master Mix for all labeling reactions was prepared:
5x Reaction Buffer 2.0 µl
10x dNTP Mix (for dATP label) 1.0 µl
[µ-32P]dATP (3,000 Ci/mmol, 10 mCi/ml; Amersham #PB10204) 3.5 µl
DTT (100 mM) 0.5 µl
MMLV Reverse Transcriptase (50 units/µl) 1.0 µl

For each reaction 8.0 µl of the Master Mix was added. The PCR thermal cycler (T3 Thermocycler, Biometra) was preheated to 70 °C. For each experimental poly A+ RNA and the Control Poly A+ RNA, the following ingredients were combined in a 0.2 ml PCR tube (Tamar, cat No. 431M):

| | |
|---|---|
| Poly A+ RNA sample | 1µg (2µl) |
| 10x CDS Primer Mix | 1 µl |

The tubes were mixed by vortexing and spun briefly in a microcentrifuge. The tubes were incubated in the preheated PCR thermal cycler at 70 °C for 2 min followed by incubation for 2 min at 50 °C. 8 µl of Master Mix was added to each reaction tube (paying attention not to remove the RNA samples from the thermal cycler for longer than is necessary to add the Master Mix), the contents of the tubes were mixed gently by pipetting and immediately returned to the thermal cycler. The tubes were incubated in the PCR thermal cycler at 50 °C for 25 min and the reaction was stopped by adding 1 µl of 10x Termination Mix (0.1 M EDTA pH 8.0; 1.0 mg/ml Glycogen [type IX from bovine liver; Sigma]).

To purify the ³²P-labeled cDNA from unincorporated ³²P -labeled nucleotides and small (<0.1kb) cDNA fragments, each reaction tube was subjected to the following procedure: for each reaction, one CHROMA SPIN-200 DEPC-H2O column was removed to completely resuspend the gel matrix. (In case of air bubbles in the column matrix the column was inverted again). The bottom cap was removed from the column followed by removing the top cap slowly. The column was placed into a 1.5-ml microcentrifuge tube refrigerator and allowed to warm up at room temperature for about 1 hr. The column was inverted to let the fluid to drain through the column until the surface of the gel beads in the column matrix could be seen. The top of the column matrix should be 1.0-ml. The collected fluid was discarded and the sample was applied carefully and slowly to the center of the gel bed's flat surface and the sample was allowed to be fully absorbed into the resin bed before proceeding to the next step.

40 µl of deionized H₂O was applied to the column and was allowed to completely drain out of the column and discarded. 250 µl of deionized H₂O was applied to the column and allowed to completely drain out of the column until no liquid remained above the gel bed, and discarded.

Four fractions were collected as follows. The column was transfer to a clean 1.5-ml microcentrifuge tube; 100 µl of deionized H₂O was added to the column and allowed to completely drain out of the column. The procedure was repeated four times. The incorporation of ³²P into the probe was checked using the Cherenkov method for scintillation counting by counting the entire sample on the tritium channel without adding scintillation cocktail to the tube.

The hybridization procedure was performed as follows, by first performing a test hybridization to a blank membrane. Before hybridizing the prepared ³²P -labeled cDNA probes to the Atlas Array, the quality of each experimental cDNA probe was checked by hybridizing it to the control (blank) nylon membrane supplied. This allowed estimation of the level of nonspecific background resulting from impurities in the RNA samples.

The nylon membrane was stored at -20 °C and was kept moistened throughout the procedure. 15 ml of ExpressHyb solution was prewarmed at 68 °C and 1.5 mg of sheared salmon testes DNA (10mg/ml; Sigma #D-7656) was heated at 95-100 °C for 5 min and then chilled quickly on ice; The heat-denatured sheared salmon testes DNA was mixed with prewarmed ExpressHyb and kept at 68 °C until use.

The Atlas Array was wet with deionized H₂O, the excess was shaken off and the Atlas Array was placed into a 10 ml hybridization solution prepared previously. Prehybridization was performed for 30 min with continuous agitation at 68 °C.

The entire pool of labeled cDNA probe, (about 200 µl, 2 and 4-10 x 10⁶ cpm, for blank and Atlas Array, respectively) was mixed with 1/10th of the reaction total volume of 10x denaturing solution (1 M NaOH, 10 mM EDTA) and incubated at 68 °C for 20 min. Then, 5 µl (1 µg/µl) of Cot-1 DNA and an equal volume (about 225 µl) of 2x neutralizing solution (1 M NaH₂PO4 [pH 7.0]) was added and incubation continued at 68 °C for 10 min.

The radioactive mixture was added to the remaining 5 ml of ExpressHyb solution; the prehybridization solution was poured out and the hybridization solution was introduced. Hybridization took place overnight with continuous agitation at 68 °C.

Solutions 1 (2x SSC; 1% SDS) and 2 (0.1x SSC; 0.5% SDS) were prewarmed at 68 °C. The hybridization solution was carefully removed and the Atlas Array was washed with 200 ml of solution 1 for 30 min with continuous agitation at 68 °C. This step was repeated four times. Two additional 30-min washes were performed with solution 2 as well.

The Atlas Array was removed from the container and excess solution was shaken off without allowing the membrane to dry. Immediately, the membrane was wrapped in plastic wrap and the Atlas Array was exposed to x-ray film at -70 °C with an intensifying screen for 3 days.

Following development of the film, the cDNA Probes were stripped from the Atlas Array and the membrane was reused. 0.5% SDS solution was heated to boil, the plastic wrap was removed from the Atlas Array and the membrane was immediately placed into the boiling solution for 10 min. The solution was removed from heat and allowed to cool for 10 min. The efficiency of the stripping was checked by exposure to x-ray film, and the stripping procedure was repeated, until radioactivity was no longer detected.

The genes on the Atlas Arrays were grouped in various functional classes:
A. Oncogenes; Tumor Suppressor genes; Cell Cycle Control Proteins.
B. Stress Response proteins; Ion Channels and Transport proteins;
   Intracellular Signal Transduction Modulators and Effectors.
C. Apoptosis genes; DNA synthesis , Repair and Recombination genes.
D. Transcription Factors, General DNA-binding proteins.
E. Receptors: Growth factors and Chemokines, Interleukins and Interferons,
   Hormones, Neurotransmitters; Cell-surface Antigens; Cell Adhesion proteins.
F. Cell-cell Communication proteins: Growth Factors, Cytokines and Chemokines; Interleukins and Interferons; Hormones.
G. Housekeeping Genes
   Negative Controls: G2-4, 9-11, 16-18.
   Blank Spots: G1, 8, 15.

Both negative controls and blank spots behaved as they should upon hybridization. Following 3 days exposure, the two identical Atlas Arrays, hybridized to the two different probes, were compared, and the genes that showed different expression intensities between the two were chosen for further investigation.

**Table 2:**

| Down-regulation of genes after treatment with Halofuginone | |
|---|---|
| Gene | Effect of Halofuginone |
| RAS-related Protein RAB-5A | -- |
| Apopain (cystein protease CPP32 isoform alpha) | - |
| Apopain (cystein protease Mch2 isoform beta) | -- |
| MUTL Protein Homolog | -- |
| UV Excision Repair Protein RAD23 | -- |
| Nuclear Factor NF90 | --- |
| Homeobox Protein HOX-D3 | -- |
| Glyceraldehyde 3-Phosphate Dehydrogenase (G3PDH) | ----- |

### The regulation of human cell interaction genes after treatment with Halofuginone

The same protocol was used as previously. Primary human fibroblasts were treated for 1hr with 10⁻⁸ M Halofuginone. The Halofuginone was previously tested at the level ofRNA and collagen α1(I) mRNA was found to be reduced upon treatment with Halofuginone. cDNA probes were prepared from either mRNA from cells treated or not treated with Halofuginone and hybridized against Clontech's Atlas cDNA Expression Arrays: Atlas Human Cell Interaction Array, cat No. 7746-1. Arrays include 265 genes known to be involved in various types of cell interactions. Following hybridization, the two membranes were compared, and genes that showed different expression intensities between the two were chosen for further investigation.

**Table 3:**

| Up-regulation of human cell interaction genes after treatment with Halofueinone | |
|---|---|
| Gene | Effect of Halofuginone |
| Laminin receptor | +++ |
| IGFBP6 | + |
| Integrin alpha 3 | + |
| TIMP-1 | ++ |
| TIMP-2 | ++ |
| CD59 | ++ |
| Nucleoside Diphosphate Kinase B | ++ |
| Transforming protein RHOA | ++ |

**Table 4:**

| Down-regulation of human cell interaction genes after treatment with Halofuginone | |
|---|---|
| Gene | Effect of Halofuginone |
| G3PDH | ----- |
| 23 kDA highly basic protein | -- |
| CD9 | -- |
| Wnt-13 | -- |
| Caveolin-1 | -- |
| rhoG | --- |

From these experimental results, Halofuginone clearly alters the regulation of a number of different genes, thereby affecting the extracellular matrix economy with regard to a large number of different gene products. However, many of these effects on different genes are presumably secondary to the main mechanism of action of Halofuginone and related molecules of this class, which includes inhibiting the expression of the collagen α1(I) gene; inhibiting collagenase type IV production; inhibiting H19 gene expression; decreasing the expression of the HSP47 gene in parallel to the inhibition of the expression of the collagen α1(I) gene; decreasing the release of the cytokines IL-1β and TNFα, while inhibiting the transcription of NF-κB; but without affecting the expression of TGFβ.

Thus, the apparent effects of Halofuginone with regard to TNF-α and NF-κB, but not TGF-β, may ultimately enable Halofuginone to have the desired inhibitory effects on the pathological processes of fibrosis, substantially without detrimentally inhibiting or downregulating the physiologically normal and desirable extracellular matrix economy.

### Example 7

### Effectors for Regulating the Extracellular Matrix Economy

As clearly demonstrated above, the extracellular matrix economy can be regulated by Halofuginone and other effectors at a number of intervention points. These intervention points enable pathological processes associated with tissue trauma, and other mechanistically related processes, to be selectively inhibited while maintaining substantially normal activity of physiologically desirable processes. As noted previously, the term "mechanistically related processes" refers to those pathological conditions which share one or more underlying mechanisms with the abnormal responses to tissue trauma. An example of such a mechanistically related process is the growth and metastasis of malignant cancer cells.

The underlying mechanism of action of Halofuginone in the inhibition of all of these pathological processes involves the inhibition of collagen type I synthesis at the molecular level, in particular by promoting *cKrox* activity and hence inhibiting the expression of collagen α1(I) gene expression, as well as by decreasing the expression of the HSP47 gene in parallel to the inhibition of the expression of the collagen α1(I) gene; decreasing the release of the cytokines IL-1β and TNFα, while inhibiting the transcription of NF-κB; but without affecting the expression of TGFβ.. The term "promotion of *cKrox* activity" includes, but is not limited to, the upregulation of *cKrox* gene expression and the enhancement of the activity of the *cKrox* protein or proteins.

Other potential molecular targets for Halofuginone include the inhibition of collagenase type IV production and the inhibition of H19 gene expression, as well as the overall regulation of ECM (extracellular matrix) deposition and remodeling, and the inhibition of neo-angiogenesis. Other mechanisms of the "extracellular matrix economy" include the induction of apoptosis and the inhibition of H19 gene expression. Clearly, the elucidation of these mechanisms and of the overall selective regulation of the extracellular matrix economy demonstrates novel and non-obvious methods for therapeutic intervention which have not been taught nor suggested by the background art.

Therefore, the present invention is also contemplated to include methods and compositions for the regulation of the extracellular matrix economy, and in particular for the promotion of *cKrox* activity and for the inhibition of the HSP47 gene in parallel to the inhibition of the expression of the collagen α1(I) gene; the decrease in the release of the cytokines IL-1β and TNFα, with the inhibition of the transcription of NF-κB; but substantially without affecting the expression of TGFβ.

These methods and compositions include the administration of an effector to a subject. The term "effector" as used herein refers to substantially any chemical compound or combination thereof which is able to regulate the extracellular matrix economy as described above. Preferably, the effector is able to promote *cKrox* activity. More preferably, such promotion is caused by enhancing the expression of the *cKrox* gene. Alternatively and preferably, the effector is able to inhibit the HSP47 gene in parallel to the inhibition of the expression of the collagen α1(I) gene; decrease the release of the cytokines IL-1β and TNFα, as well as inhibiting the transcription of NF-κB; yet is able to exert these effects substantially without affecting the expression of TGFβ. More preferably, the effector is capable of inducing this constellation of effects as a group.

In preferred embodiments of compositions of the present invention, these compositions include a quinazolinone derivative as the effector. In further preferred embodiments of the present invention, these compositions include Halofuginone as the effector.

The effector of the present invention can be administered to a subject in a number of ways, which are well known in the art. Hereinafter, the term "subject" refers to the human or lower animal to whom the effector was administered. For example, administration may be done topically (including opthalmically, vaginally, rectally, intranasally), orally, or parenterally, for example by intravenous drip or intraperitoneal, subcutaneous, or intramuscular injection.

Formulations for topical administration may include but are not limited to lotions, ointments, gels, creams, suppositories, drops, liquids, sprays and powders. Conventional pharmaceutical carriers, aqueous, powder or oily bases, thickeners and the like may be necessary or desirable.

Compositions for oral administration include powders or granules, suspensions or solutions in water or non-aqueous media, sachets, capsules or tablets. Thickeners, diluents, flavorings, dispersing aids, emulsifiers or binders may be desirable.

Formulations for parenteral administration may include sterile aqueous solutions which may also contain buffers, diluents and other suitable additives.

Dosing is dependent on the severity of the symptoms and on the responsiveness of the subject to the effector. Persons of ordinary skill in the art can easily determine optimum dosages, dosing methodologies and repetition rates.

The following example is an illustration only of a method of regulating the extracellular economy with an effector such as Halofuginone in order to treat a pathological condition associated with tissue trauma or a cnechanistically related condition.

### Example 8

### Method of Treatment of Cardiac Fibrosis

As noted above, Halofuginone has been shown to be an effective inhibitor of cardiac fibrosis. The following example is an illustration only of a method of treating cardiac fibrosis with Halofuginone.

The method includes the step of using Halofuginone, in a pharmaceutically acceptable carrier as described in Example 7 above, for adminishation, to a subject to be treated. Halofuginone is to be administered according to an effective dosing methodology, preferably until a predefined endpoint is reached, such as the absence of further progression of cardiac fibrosis in the subject, the inhibition of cardiac fibrosis or the prevention of the formation of cardiac fibrosis.

## Claims

1. A method of manufacturing a medicament for treating cardiac fibrosis, comprising the step of placing a pharmaceutically effective amount of a compound in a pharmaceutically acceptable carrier, the compound being a member of a group having a formula: 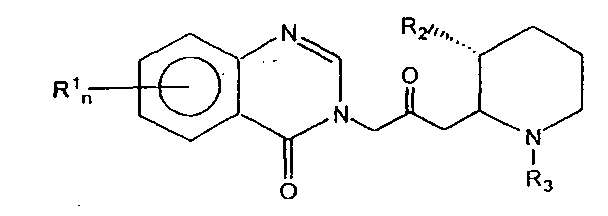 wherein:
R₁ is a member of the group consisting of hydrogen, halogen, nitro, benzo, lower alkyl, phenyl and lower alkoxy; R₂ is a member of the group consisting of hydroxy, acetoxy, and lower alkoxy; R₃ is a member of the group consisting of hydrogen and lower alkenoxy-carbonyl; and *n* is either I or 2; and pharmaceutically acceptable salts thereof.

2. Use of a compound having a formula: 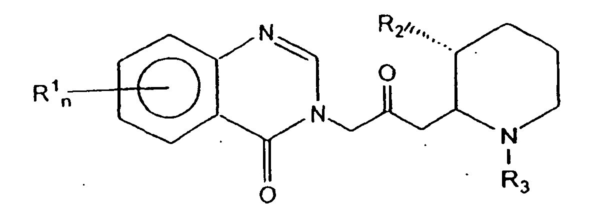 wherein:
R₁ is a member of the group consisting of hydrogen, halogen, nitro, benzo, lower alkyl, phenyl, and lower alkoxy; R₂ is a member of the group consisting of hydroxy, acetoxy and lower alkoxy; R₃ is a member of the group consisting of hydrogen and lower alkenoxy-carbonyl; and *n* is either 1 or 2; or of a pharmaceutically acceptable salt of such a compound; for the manufacture of a medicament for treating cardiac fibrosis.

3. A method of manufacturing a medicament for preventing cardiac fibrosis, comprising the step of placing a pharmaceutically effective amount of a compound in a pharmaceutically acceptable carrier, the compound being a member of a group having a formula; 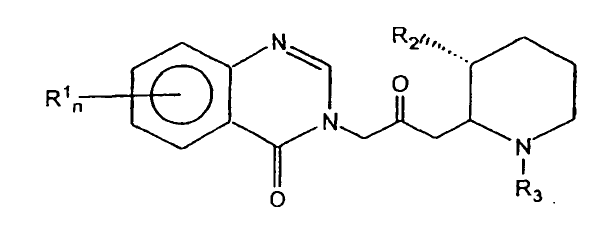 wherein:
R₁ is a member of the group consisting of hydrogen, halogen, nitro, benzo, lower alkyl, phenyl, and lower alkoxy; R₂ is a member of the group consisting of hydroxy, acetoxy, and lower alkoxy; R₃ is a member of the group consisting of hydrogen and lower alkenoxy-carbonyl; and *n* is either 1 or 2; and pharmaceutically acceptable salts thereof.

4. Use of a compound having a formula: 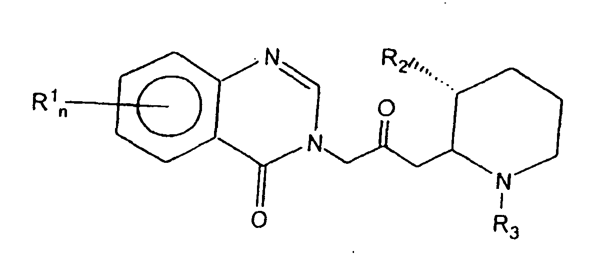 wherein:
R₁ is a member of the group consisting of hydrogen, halogen, nitro, benzo, lower alkyl, phenyl, and lower alkoxy; R₂ is a member of the group consisting of hydroxy, acetoxy and lower alkoxy, R₃ is a member of the group consisting of hydrogen and lower alkenoxy-carbonyl; and *n* is either I or 2; or of a pharmaceutically acceptable salt of such a compound; for the manufacture of a medicament for preventing cardiac fibrosis.

## Patentansprüche

1. Ein Verfahren zur Herstellung eines Medikaments zur Behandlung einer Herzfibrose, das den Schritt der Platzierung einer pharmazeutisch wirksamen Menge einer Verbindung in ein pharmazeutisch verträgliches Trägermittel umfasst, wobei die Verbindung ein Mitglied aus einer Gruppe mit der folgenden Formel ist: 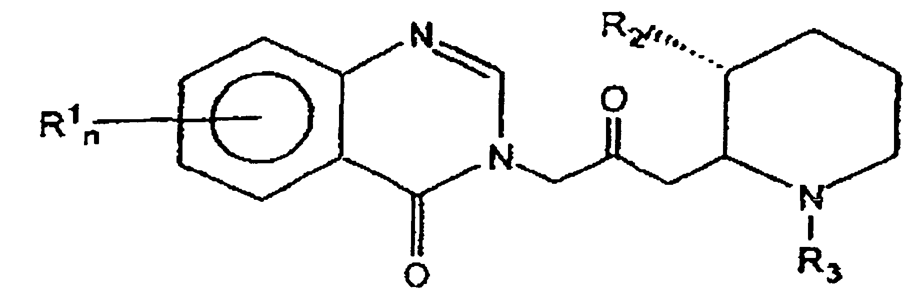 worin R₁ ein Mitglied aus der Gruppe bestehend aus Wasserstoff, Halogen, Nitro, Benzo, niederem Alkyl, Phenyl und niederem Alkoxy ist; R₂ ein Mitglied der Gruppe bestehend aus Hydroxy, Acetoxy und niederem Alkoxy ist; R₃ ein Mitglied aus der Gruppe bestehend aus Wasserstoff und niederem Alkenoxycarbonyl ist; und n entweder 1 oder 2 ist; und pharmazeutisch verträgliche Salze davon.

2. Verwendung einer Verbindung mit einer Formel: 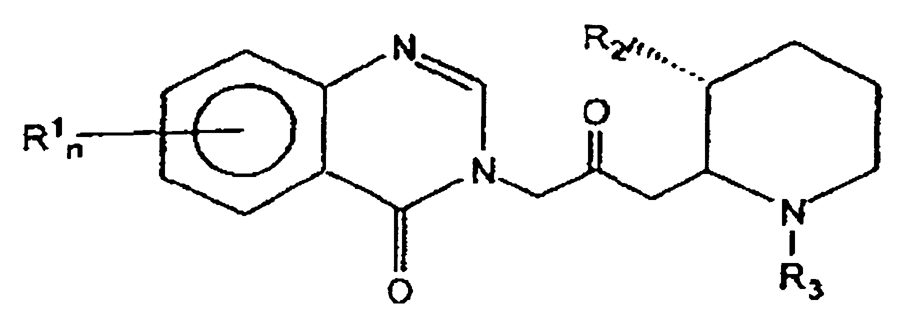 worin R₁ ein Mitglied aus der Gruppe bestehend aus Wasserstoff, Halogen, Nitro, Benzo, niederem Alkyl, Phenyl und niederem Alkoxy ist; R₂ ein Mitglied der Gruppe bestehend aus Hydroxy, Acetoxy und niederem Alkoxy ist; R₃ ein Mitglied aus der Gruppe bestehend aus Wasserstoff und niederem Alkenoxycarbonyl ist; und *n* entweder 1 oder 2 ist; oder ein pharmazeutisch verträgliches Salz einer solchen Verbindung, zur Herstellung eines Medikaments zur Behandlung einer Herzfibrose.

3. Ein Verfahren zur Herstellung eines Medikaments zur Verhinderung einer Herzfibrose, das den Schritt der Platzierung einer pharmazeutisch wirksamen Menge einer Verbindung in ein pharmazeutisch verträgliches Trägermittel umfasst, wobei die Verbindung ein Mitglied aus einer Gruppe mit der folgenden Formel ist: 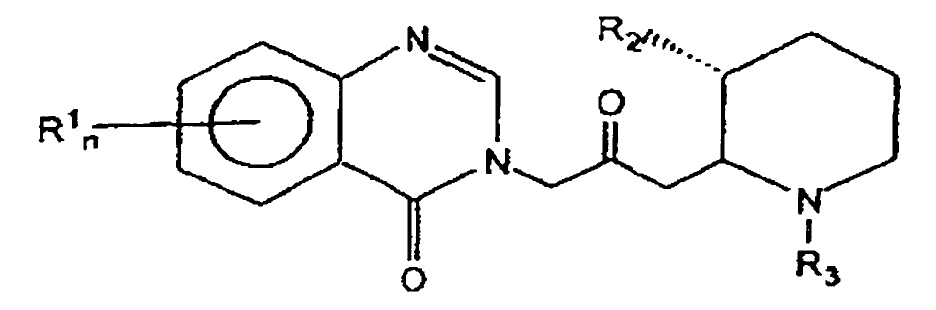 worin R₁ ein Mitglied aus der Gruppe bestehend aus Wasserstoff, Halogen, Nitro, Benzo, niederem Alkyl, Phenyl und niederem Alkoxy ist; R₂ ein Mitglied der Gruppe bestehend aus Hydroxy, Acetoxy und niederem Alkoxy ist; R₃ ein Mitglied aus der Gruppe bestehend aus Wasserstoff und niederem Alkenoxycarbonyl ist; und *n* entweder 1 oder 2 ist; und pharmazeutisch verträgliche Salze davon.

4. Verwendung einer Verbindung mit einer Formel: 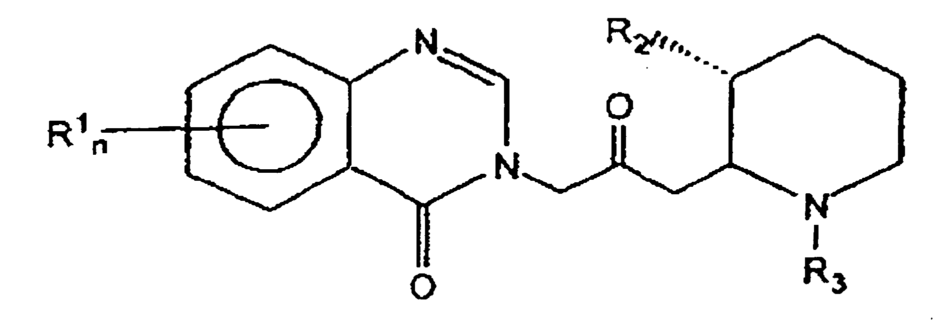 worin R₁ ein Mitglied aus der Gruppe bestehend aus Wasserstoff, Halogen, Nitro, Benzo, niederem Alkyl, Phenyl und niederem Alkoxy ist; R₂ ein Mitglied der Gruppe bestehend aus Hydroxy, Acetoxy und niederem Alkoxy ist; R₃ ein Mitglied aus der Gruppe bestehend aus Wasserstoff und niederem Alkenoxycarbonyl ist; und n entweder 1 oder 2 ist; oder ein pharmazeutisch verträgliches Salz einer solchen Verbindung, zur Herstellung eines Medikaments zur Verhinderung einer Herzfibrose.

## Revendications

1. Procédé de fabrication d'un médicament pour traiter une fibrose cardiaque, comprenant l'étape de mise en place d'une quantité pharmaceutiquement efficace d'un composé dans un véhicule pharmaceutiquement acceptable, le composé étant un élément d'un groupe ayant la formule suivante : 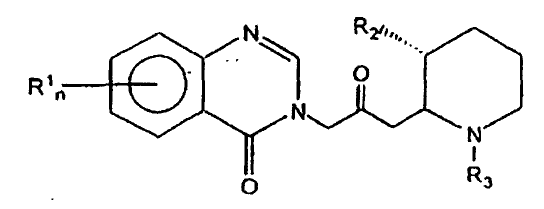 dans laquelle :
R₁ est un élément du groupe constitué de l'hydrogène, d'un halogène et de groupements nitro, benzo, alkyle inférieur, phényle et alcoxy inférieur ; R₂ est un élément du groupe constitué de groupements hydroxy, acétoxy et alcoxy inférieur ; R₃ est un élément du groupe constitué de l'hydrogène et d'un groupement alcénoxy(inférieur)carbonyle ; et n vaut 1 ou 2 ; et de ses sels pharmaceutiquement acceptables.

2. Utilisation d'un composé ayant la formule : 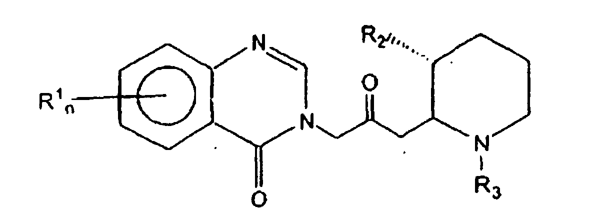 dans laquelle :
R₁ est un élément du groupe constitué de l'hydrogène, d'un halogène et de groupements nitro, benzo, alkyle inférieur, phényle et alcoxy inférieur ; R₂ est un élément du groupe constitué de groupements hydroxy, acétoxy et alcoxy inférieur; R₃ est un élément du groupe constitué de l'hydrogène et d'un groupement alcénoxy(inférieur)carbonyle ; et n vaut 1 ou 2 ; ou d'un sel pharmaceutiquement acceptable de ce composé, pour la fabrication d'un médicament destiné à traiter une fibrose cardiaque.

3. Procédé de fabrication d'un médicament pour prévenir une fibrose cardiaque, comprenant l'étape de mise en place d'une quantité pharmaceutiquement efficace d'un composé dans un véhicule pharmaceutiquement acceptable, le composé étant un élément d'un groupe ayant la formule suivante : 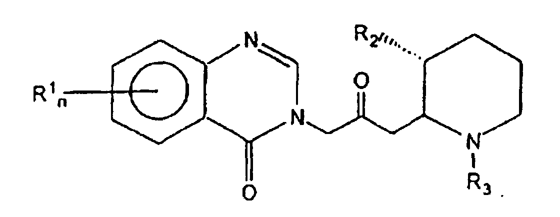 dans laquelle :
R₁ est un élément du groupe constitué de l'hydrogène, d'un halogène et de groupements nitro, benzo, alkyle inférieur, phényle et alcoxy inférieur ; R₂ est un élément du groupe constitué de groupements hydroxy, acétoxy et alcoxy inférieur ; R₃ est un élément du groupe constitué de l'hydrogène et d'un groupement alcénoxy(inférieur)carbonyle ; et n vaut 1 ou 2 ; et de ses sels pharmaceutiquement acceptables.

4. Utilisation d'un composé ayant la formule suivante : 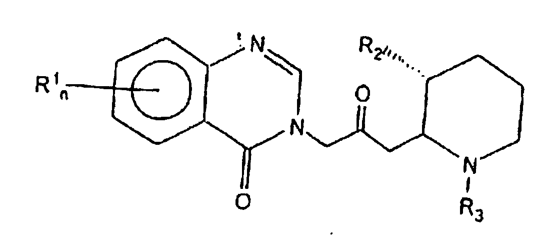 dans laquelle :
R₁ est un élément du groupe constitué de l'hydrogène, d'un halogène et de groupements nitro, benzo, alkyle inférieur, phényle et alcoxy inférieur ; R₂ est un élément du groupe constitué de groupements hydroxy, acétoxy et alcoxy inférieur; R₃ est un élément du groupe constitué de l'hydrogène et d'un groupement alcénoxy(inférieur)carbonyle ; et n vaut 1 ou 2 ; ou d'un sel pharmaceutiquement acceptable de ce composé, pour la fabrication d'un médicament destiné à prévenir une fibrose cardiaque.
